# NOUVEAU FASCICULE DE BREVET EUROPEEN

(11) **EP 0 919 627 B2**
(45) Date de publication et mention de la décision concernant l'opposition: **14.03.2007**
(45) Mention de la délivrance du brevet: 20.09.2000
(21) Numéro de dépôt: 98124039.3
(22) Date de dépôt: 27.05.1994
(51) Int. Cl.: C12N 5/16, C12N 15/861, C12N 15/34, A61K 48/00, C12N 7/04, A61K 39/235

(54) **Nouveaux adenovirus défectifs et lignées de complémentation correspondantes**
Neue defektive Adenoviren und passende transkomplementierende Zelllinien
New defective adenovirus and corresponding transcomplementing celllines

(30) Priorité: 28.05.1993 FR 9306482
(43) Date de publication de la demande: 02.06.1999
(62) Demande divisionnaire de: 94917063.3
(73) Titulaire: TRANSGENE S.A., 67082 Strasbourg Cédex (FR)
(72) Inventeur: Imler, Jean-Luc, 67000 Strasbourg (FR); Mehtali, Majid, 67400 Illkirch-Graffenstaden (FR); Pavirani, Andrea, 67000 Strasbourg (FR)
(74) Mandataire: Vossius & Partner

(56) Documents cités:
- WO-A-91/11525
- KETNER G ET AL: "COMPLEMENTATION OF ADENOVIRUS E4 MUTANTS BY TRANSIENT EXPRESSION OF E4 CDNA DELETION PLASMIDS" NUCLEIC ACIDS RESEARCH, vol. 17, no. 8, 1 janvier 1989, pages 3037-3048, XP002004339
- WEINBERG D H ET AL: "A CELL LINE THAT SUPPORTS THE GROWTH OF A DEFECTIVE EARLY REGION 4 DELETION MUTANT OF HUMAN ADENOVIRUS TYPE 2" PROCEEDINGS OF THE NATIONAL ACADEMY OF SCIENCES OF USA, vol. 80, 1 septembre 1983, pages 5383-5386, XP000672082

### LISTE DE SEQUENCES

(1) INFORMATION GENERALE:
   (i) DEPOSANT:
      (A) NOM: TRANSGENE
      (B) RUE: 11, rue de Molsheim
      (C) VILLE: STRASBOURG
      (E) PAYS: FRANCE
      (F) CODE POSTAL: 67082
      (G) TELEPHONE: (33) 88.27.91.00
      (H) TELECOPIE: (33) 88.22.58.07
   (ii) TITRE DE L'INVENTION: Nouveaux adenovirus défectifs et lignées de complémentation correspondantes
   (iii) NOMBRE DE SEQUENCES: 42
   (iv) FORME LISIBLE PAR ORDINATEUR:
      (A) TYPE DE SUPPORT: Tape
      (B) ORDINATEUR: IBM PC compatible

      (C) SYSTEME D' EXPLOITATION: PC-DOS/MS-DOS
      (D) LOGICIEL: Patentln Release #1.0, Version #1.25 (OEB)
(2) INFORMATION POUR LA SEQ ID NO: 1:
   (il CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      é (A) ORGANISME: Oligonucléotide de synthèse (OTG4174)
   (xi) DESCRIPTION DE LA SEQUENCE: SEQ ID NO: 1:
      GTGACGTCTT TGGTGTTTTC GCGGGAAAAC 30
(2) INFORMATION POUR LA SEQ ID NO: 2:
   (i) CARACTERlSTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG4173)
   (xi) DESCRIPTION DE LA SEQUENCE: SEQ ID NO: 2:
      ACCGAGTAAG ATTTGTCTAG GGCCGCGGGG 30
(2) INFORMATION POUR LA SEQ ID NO: 3:
   (i) CARACTERISTIQUES DE LA SEQUENCE:
      (A) LONGUEUR: 33 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG4191)
   (xi) DESCRIPTION DE LA SEQUENCE: SEQ ID NO: 3:
      GGCCATGGTC GCGGGAAAGG GACTTTGACC GTT 33
(2) INFORMATION POUR LA SEQ ID NO: 4:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5021)
   (xi) DESCRIPTION DE LA SEQUENCE: SEQ ID NO: 4:
      GAACGGATCC CCAGACTCTG TTTGGATTTG G 31
(2) INFORMATION POUR LA SEQ ID NO: 5:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE NON
   (iii) ANTI-SENS: OUI
   (vi) ORIGINE:
      (A) ORGANISME: Oligonucléotide de synthèse (OTG5157)
   (xi) DESCRIPTION DE LA SEQUENCE: SEQ ID NO: 5:
      CCAGAAATAT CTTCGCCCAG GCCGCCGCCC 30
(2) INFORMATION POUR LA SEQ ID NO: 6:
   (i) CARACTERISTIQUES DE LA SEQUENCE:
      (A) LONGUEUR 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5564)
   (xi) DESCRIPTION DE LA SEQUENCE: SEQ ID NO: 6:
      GATCCGATAT CCCGTTAACC 20
(2) INFORMATION POUR LA SEQ ID NO: 7:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5565)
   (xi) DESCRIPTION DE LA SEQUENCE: SEQ ID NO: 7:
      GATCGGTTAA CGGGATATCG 20
(2) INFORMATION POUR LA SEQ ID NO: 8:
   (i) CARACTERISTIQUES DE LA SEQUENCE:
      (A) LONGUEUR: 47 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5892)
   (xi) DESCRIPTION DE LA SEQUENCE: SEQ ID NO: 8:
      GTCGTAGGAT CCAGCTGCTC CCTGCTTGTG TGTTGGAGGT CGCTGAG 47
(2) INFORMATION POUR LA SEQ ID NO: 9:
   (i) CARACTERISTIQUES DE LA SEQUENCE:
      (A) LONGUEUR: 47 paires de bases
      (B) TYPE: acide nucléique
      (C) NOMBRE DE BRINS: simple.
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5893)
   (xi) DESCRIPTION DE LA SEQUENCE: SEQ ID NO: 9:
      GTAGCTGACG TCCCAGGTGC ACACCAATGT GGTGAATGGT CAAATGG 47
(2) INFORMATION POUR LA SEQ ID NO: 10:
   (i) CARACTERISTIQUES DE LA SEQUENCE:
      (A) LONGUEUR: 46 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5920)
   (xi) DESCRIPTION DE LA SEQUENCE: SEQ ID NO: 10:
      ACGGTAGGAT CCGACGTCGG TGAGCTCCTC GCTTGGTCTC CGTCCG 46
(2) INFORMATION POUR LA SEQ ID NO: 11:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vl) ORIGINE:
      (A) ORGANISME: Oligonucléotide de synthèse (OTG5891)
   (xi) DESCRIPTION DE LA SEQUENCE: SEQ ID NO: 11:
      CAACCCCGAT TCTAGAGAAA CCTG 24
(2) INFORMATION POUR LA SEQ ID NO: 12
   (i) CARACTERISTIQUES DE LA SEQUENCE:
      (A) LONGUEUR: 35 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6079)
   (xi) DESCRIPTION DE LA SEQUENCE: SEQ ID NO: 12:
      GCGCAGTTGC TCTGCGGATC CACTTAACAT TCAGT 35
(2) INFORMATION POUR LA SEQ ID NO: 13:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6080)
   (xi) DESCRIPTION DE LA SEQUENCE: SEQ ID NO:13:
      TAAAAGTACC AGGTAAGGAT CCCCTTGGTT TGCTTGGG 38
(2) INFORMATION POUR LA SEQ ID NO:14:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG6064)
   (xi) DESCRIPTION DE LA SEQUENCE: SEQ ID NO:14:
      GAAACCGAAT TCTCTTGGAA C 21
(2) INFORMATION POUR LA SEQ ID NO: 15:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6065)
   (xi) DESCRIPTION DE LA SEQUENCE: SEQ ID NO: 15:
      ACGAATGCAG CTCTCCACTT AACATTCAGT CG 32
(2) INFORMATION POUR LA SEQ ID NO: 16:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5481)
   (xi) DESCRIPTION DE LA SEQUENCE: SEQ ID NO:16:
      CAGTGAATTC ATCATCAATA ATATACC 27
(2) INFORMATION POUR LA SEQ ID NO: 17:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5482)
   (xi) DESCRIPTION DE LA SEQUENCE: SEQ ID NO: 17:
      AAACTGGTCA CCGTGATTAA AAAG 24
(2) INFORMATION POUR LA SEQ ID NO: 18:
   (i) CARACTERISTIQUES DE LA SEQUENCE
      (A) LONGUEUR: 25 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5455)
   (xi) DESCRIPTION DE LA SEQUENCE: SEQ ID NO: 18:
      ATCGGAATTC AAGATGATTA GGTAC 25
(2) INFORMATION POUR LA SEQ ID NO: 19:
   (i) CARACTERISTIQUES DE LA SEQUENCE:
      (A) LONGUEUR: 28 paires de bases
      (B) TYPE: acide nucléique
      (C) NOMBRE DE BAINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5456)
   (xi) DESCRIPTION DE LA SEQUENCE: SEQ ID NO: 19:
      ATCGTCTAGA TTAAGGCATT TTCTTTTC 28
(2) INFORMATION POUR LA SEQ ID NO: 20:
   (i) CARACTERISTIQUES DE LA SEQUENCE:
      (A) LONGUEUR: 18 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5728)
   (xi) DESCRIPTION DE LA SEQUENCE: SEQ ID NO: 20:
      TGTAGCAGGA GGACTAAG 18
(2) INFORMATION POUR LA SEQ ID NO: 21:
   (I) CARACTERISTIQUES DE LA SEQUENCE:
      (A) LONGUEUR: 39 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5729)
   (xi) DESCRIPTION DE LA SEQUENCE: SEQ ID NO: 21:
      CCGCATTAAT TAACCGCGAC AAACGATTCT TTATTCTTG 39
(2) INFORMATION POUR LA SEQ ID NO: 22:
   (i) CARACTERISTIQUES DE LA SEQUENCE:
      (A) LONGUEUR: 36 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléolide de synthèse (5730)
   (xi) DESCRIPTION DE LA SEQUENCE: SEQ ID NO: 22:
      CGCGGTTAAT TAATGCGGTA AAACCTACGT CACCCG 36
(2) INFORMATION POUR LA SEQ ID NO: 23:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG6060)
   (xi) DESCRIPTION DE LA SEQUENCE: SEQ ID NO: 23:
      AATAAAAGAT CATTATTTTC ATTAGAACTG 30
(2) INFORMATION POUR LA SEQ ID NO: 24:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG6061)
   (xi) DESCRIPTION DE LA SEQUENCE: SEQ ID NO: 24:
      TGTGTTGGTT TTTTGTGTGT TAAT 24
(2) INFORMATION POUR LA SEQ ID NO: 25:
   (i) CARACTERISTIQUES DE LA SEQUENCE:
      (A) LONGUEUR: 30 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6062)
   (xi) DESCRIPTION DE LA SEQUENCE: SEQ ID NO: 25:
      TAACACACAA AAAACCAACA CACAGTTCTA 30
(2) INFORMATION POUR LA SEQ ID NO: 26:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6063)
   (xi) DESCRIPTION DE LA SEQUENCE: SEQ ID NO: 26:
      ATGAAAATAA TGATCTTTTA TTAT 24
(2) INFORMATION POUR LA SEQ ID NO: 27:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG4564)
   (xl) DESCRIPTION DE LA SEQUENCE: SEQ ID NO: 27:
      TCCGTGAATT CTAGTAGTGT GGCGGAAGTG TG 32
(2) INFORMATION POUR LA SEQ ID NO: 28:
   (i) CARACTERISTIQUES DE LA SEQUENCE:
      (A) LONGUEUR: 23 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG4565)
   (xi) DESCRIPTION DE LA SEQUENCE: SEQ ID NO: 28:
      TCCAGTCCGG AGACCGGGC GCC 23
(2) INFORMATION POUR LA SEQ ID NO: 29:
   (i) CARACTERISTIQUES DE LA SEQUENCE:
      (A) LONGUEUR: 28 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5013)
   (xi) DESCRIPTION DE LA SEQUENCE: SEQ ID NO: 29:
      TAACCTGCAG GAGTGCCAGC GAGTAGAG 28
(2) INFORMATION POUR LA SEQ ID NO: 30:
   (i) CARACTERISTIQUES DE LA SEQUENCE:
      (A) LONGUEUR: 21 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonuclotide de synthèse (OTG5015)
   (xi) DESCRIPTION DE LA SEQUENCE: SEQ ID NO: 30:
      CAACGCGCAT GCCCCCATGG G 21
(2) INFORMATION POUR LA SEQ ID NO: 31:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5014)
   (xi) DESCRIPTION DE LA SEQUENCE: SEQ ID NO: 31:
      TAGGAGATCT GTTTTAAACC GCATTGGGAG G 31
(2) INFORMATION POUR LA SEQ ID NO: 32:
   (i) CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse
   (xi) DESCRIPTION DE LA SEQUENCE: SEQ ID NO: 32:
      CGGAGTACTG TCCTCCGCGG ACTACTGTCC TCCG 34
(2) INFORMATION POUR LA SEQ ID NO: 33:
   (il CARACTERISTIQUES DE LA SEQUENCE:
      (A) LONGUEUR: 34 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse
   (xi) DESCRIPTION DE LA SEQUENCE: SEQ ID NO: 33:
      CGGAGGACAG TACTCCGCGG AGGACAGTAC TCCG 34
(2) INFORMATION POUR LA SEQ ID NO: 34:
   (i) CARACTERISTIQUES DE LA SEQUENCE:
      (A) LONGUEUR: 16 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5039)
   (xi) DESCRIPTION DE LA SEQUENCE: SEQ ID NO: 34:
      TGCTGGATAT CAGTCA 16
(2) INFORMATION POUR LA SEQ ID NO: 35:
   (i) CARACTERISTIQUES DE LA SEQUENCE:
      (A) LONGUEUR: 24 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5040)
   (xi) DESCRIPTION DE LA SEQUENCE: SEQ ID NO:35:
      GATCTGACTG ATATCCAGCA TGCA 24
(2) INFORMATION POUR LA SEQ ID NO:36:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5024)
   (xi) DESCRIPTION DE LA SEQUENCE: SEQ ID NO: 36:
      CTCCTGCCTA GGCAAAATAG 20
(2) INFORMATION POUR LA SEQ ID NO: 37:
   (i) CARACTERISTIQUES DE LA SEQUENCE:
      (A) LONGUEUR: 32 paires de bases
      (B) TYPE: acide nucléique
      (C) NOMBRE DE BRINS: simple
      (D) CONFIGURATION : linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG5025)
   (xi) DESCRIPTION DE LA SEQUENCE: SEQ ID NO: 37:
      GCAGATGGAT CCGGGCGGAG TAACTTGTAT GT 32
(2) INFORMATION POUR LA SEQ ID NO: 38:
   (i) CARACTERISTIQUES DE LA SEQUENCE:
      (A) LONGUEUR: 31 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5078)
   (xi) DESCRIPTION DE LA SEQUENCE: SEQ ID NO: 38:
      CTCGCGGATC CGTTATGTTT CAACGTGTTT A 31
(2) INFORMATION POUR LA SEQ ID NO: 39:
   (i) CARACTERISTIQUES DE LA SEQUENCE:
      (A) LONGUEUR: 20 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonuléotide de synthèse (OTG5079)
   (xi) DESCRIPTION DE LA SEQUENCE: SEQ ID NO: 39:
      ACATGAACTT AAGCGAGCTG 20
(2) INFORMATION POUR LA SEQ ID NO: 40:
   (i) CARACTERISTIQUES DE LA SEQUENCE:
      (A) LONGUEUR: 38 paires de bases
      (B) TYPE: acide nucléique
      (C) NOMBRE DE BRINS: simple
      (D) CONFIGURATION: linéaire
   (il) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG5991)
   (xi) DESCRIPTION DÉ LA SEQUENCE: SEQ ID NO: 40:
      CACGGCACCA GCTCAAGTTA ACGGATCCAT CTGCGGGT 38
(2) INFORMATION POUR LA SEQ ID NO: 41:
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: NON
   (vi) ORIGINE:
      (A) ORGANISME: Oligonucléotide de synthèse (OTG6141)
   (xi) DESCRIPTION DE LA SEQUENCE: SEQ ID NO: 41:
      GATCCTGTGT GTTGGTTTTT TGTGTGC 27
(2) INFORMATION POUR LA SEQ ID NO: 42
   (i) CARACTERISTIQUES DE LA SEQUENCE:
      (A) LONGUEUR: 27 paires de bases
      (B) TYPE: acide nucléique
      (C) NOMBRE DE BRINS: simple
      (D) CONFIGURATION: linéaire
   (ii) TYPE DE MOLECULE: ADN (génomique)
   (iii) HYPOTHETIQUE: NON
   (iii) ANTI-SENS: OUI
   (vi) ORIGINE:
      (A) ORGANISME: Oligonucléotide de synthèse (OTG6142)
   (xi) DESCRIPTION DE LA SEQUENCE: SEQ ID NO: 42:
      GATCGCACAC AAAAAACCAA CACACAG 27

## Description

L'invention a pour objet de nouvelles lignées cellulaires de complémentation complémentant *en trans* les fonctions virales essentielles qui ont été délétées du génome de vecteurs adénoviraux défectifs permettant le transfert et l'expression de gènes d'intérêt dans une cellule ou un organisme eucaryote hôte. L'invention présente un intérêt tout particulier pour des perspectives de thérapie génique, notamment chez l'homme.

Les adénovirus sont des virus à ADN qui présentent un large spectre d'hôte. Ils ont été mis en évidence dans de nombreuses espèces animales et de nombreux types cellulaires. Il existe plusieurs sérotypes qui diffèrent notamment au niveau de la séquence de leurs génomes. La plupart des adénovirus humains sont peu pathogènes et ne produisent généralement que des symptômes bénins.

L'adénovirus pénètre dans la cellule hôte permissive par l'intermédiaire d'un récepteur spécifique, puis il est internalisé et passe dans des endosomes. Leur acidification contribue à un changement de conformation du virus et à sa sortie dans le cytoplasme. Puis, l'ADN viral associé à certaines protéines virales nécessaires aux premières étapes du cycle réplicatif, pénètre dans le noyau des cellules infectées où sa transcription est initiée par des enzymes cellulaires. La réplication de l'ADN adénoviral a lieu dans le noyau des cellules infectées et ne nécessite pas la réplication cellulaire. L'assemblage des nouveaux virions prend également place dans le noyau. Dans un premier temps, les protéines virales s'assemblent de manière à former des capsides vides de structure icosaedrique, dans lesquelles l'ADN adénoviral est ensuite encapsidé. Les particules virales ou virions sont libérés des cellules infectées et sont susceptibles d'infecter d'autres cellules permissives.

Le cycle infectieux de l'adénovirus s'effectue en 2 étapes :
- la phase précoce qui précède l'initiation de la réplication du génome adénoviral et qui permet la production des protéines régulatrices intervenant au niveau de la réplication et de la transcription de l'ADN viral, et
- la phase tardive qui conduit à la synthèse des protéines structurales.

D'une manière générale, le génome adénoviral est constitué d'une molécule d'ADN linéaire, bicaténaire et d'environ 36kb de long qui contient les séquences codant pour plus de 30 protéines. A chacune de ses extrémités, est présente une courte séquence de 100 à 150 nucléotides selon les sérotypes, inversée et désignée ITR (Inverted Terminal Repeat). Les ITRs sont impliqués dans la réplication du génome adénoviral. La région d'encapsidation, d'environ 300 nucléotides, est située à l'extrémité 5' du génome juste après l'ITR 5'.

Les gènes précoces sont répartis en 4 régions qui sont dispersées dans le génome adénoviral, désignées E1 à E4 (E pour "Early" signifiant précoce en anglais). Les régions précoces comprennent au moins six unités transcriptionnelles qui possèdent leurs propres promoteurs. L'expression des gènes précoces est elle même régulée, certains gènes étant exprimés avant d'autres. Trois régions, respectivement E1, E2 et E4, sont essentielles à la replication virale. Ainsi, si un adénovirus est défectif pour l'une de ces fonctions, c'est à dire s'il ne peut pas produire au moins une protéine codée par l'une de ces régions, celle-ci devra lui être fournie *en trans.*

La région précoce E1 est située à l'extrémité 5' du génome adénoviral et contient 2 unités de transcription virales, respectivement E1A et E1B. Cette région code pour des protéines qui interviennent très précocement dans le cycle viral et sont essentielles à l'expression de presque tous les autres gènes de l'adénovirus. En particulier, l'unité de transcription E1A code pour une protéine trans-activatrice de la transcription des autres gènes viraux, qui induit la transcription à partir des promoteurs des régions E1B, E2A, E2B et E4.

Les produits de la région E2, laquelle comprend également deux unités de transcription E2A et E2B, sont directement impliqués dans la réplication de l'ADN viral. Cette région gouverne notamment la synthèse d'une protéine de 72kDa, qui présente une forte affinité pour l'ADN simple brin et d'une ADN polymérase.

La région E3 n'est pas essentielle à la réplication du virus. Elle code pour au moins six protéines qui seraient responsables de l'inhibition de la réponse immune de l'hôte vis à vis d'une infection par adénovirus. En particulier, la glycoprotéine gp19kDa empêcherait la réponse CTL, responsable de la cytolyse des cellules infectées par les cellules T cytotoxiques de l'hôte.

La région E4 est située à l'extrémité 3' du génome adénoviral. Elle code pour de nombreux polypeptides qui sont impliqués dans l'expression des gènes tardifs, la stabilité des messagers (ARNm) tardifs, le passage de la phase précoce à la phase tardive ainsi que l'inhibition de la synthèse protéique cellulaire.

Une fois la réplication de l'ADN viral initiée, la transcription des gènes tardifs débute. Ceux-ci occupent la majorité du génome adénoviral et recouvrent en partie les unités de transcription des gènes précoces. Mais ils sont transcrits à partir de promoteurs différents et selon un mode d'épissage alternatif, de sorte que les mêmes séquences sont utilisées à des fins différentes. La plupart des gènes tardifs sont transcrits à partir du promoteur majeur tardif MLP (Major Late Promoter). Ce promoteur permet la synthèse d'un long transcrit primaire qui est ensuite maturé en une vingtaine d'ARN messagers (ARNm) à partir desquels sont produites les protéines capsidaires du virion. Le gène codant pour la protéine structurale IX composant la capside est situé à l'extrémité 5' du génome-adénoviral et recouvre la région E1B à son extrémité 3'. L'unité transcriptionnelle de la protéine IX utilise le même signal de terminaison de la transcription que l'unité transcriptionnelle E1B.

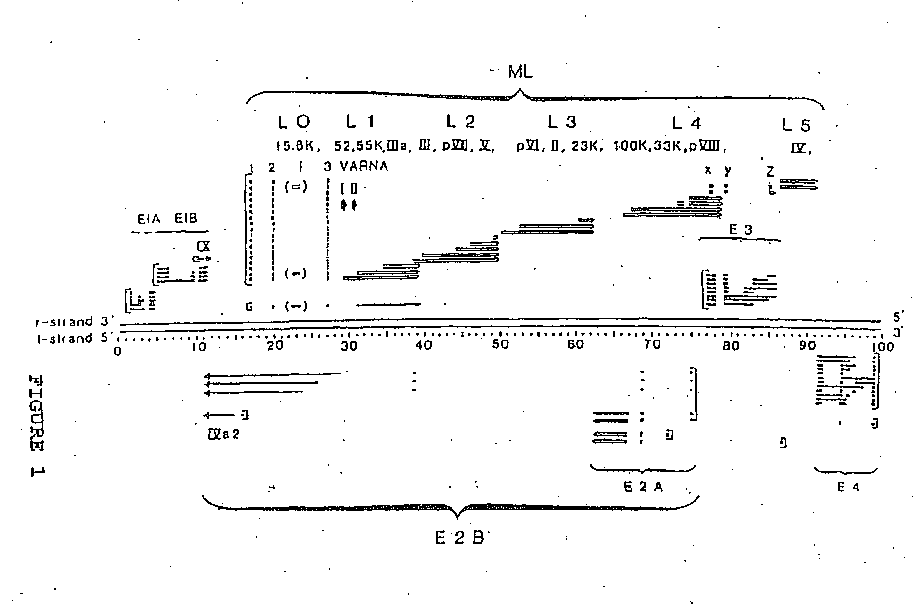

Un certain nombre d'adénovirus sont maintenant bien caractérisés génétiquement et biochimiquement. Tel est le cas de l'adénovirus humain de type 5 (Ad5) dont la séquence est divulguée dans la banque de donnée Genebank sous la référence M73260. Les différents gènes ont pu être localisés précisément sur le génome adénoviral qui comprend de 5' vers 3' l'ITR 5' de 103 bp suivi de la région d'encapsidation (Hearing et al., 1987, J. Virol., 61, 2555-2558) d'environ 300 pb, puis des régions précoces et tardives dont l'emplacement est schématiquement représenté dans la Figure 1, et enfin de l'ITR 3'.

Il ressort de ce qui précède que les adénovirus possèdent des caractéristiques intéressantes qui font d'eux des vecteurs de choix pour le transfert de gènes d'intérêt. De nombreux adénovirus recombinants sont décrits dans la littérature (Rosenfeld et al., 1991, Science, *252*, 431-434 ; Rosenfeld et al., 1992, Cell, *68*,143-155). D'une manière générale, ils dérivent de l'Ad5 et sont défectifs pour la fonction E1, afin d'éviter leur dissémination dans l'environnement et l'organisme hôte. En outre, la région E3 non-essentielle peut également être délétée. Les séquences exogènes sont intégrées à la place de la région E1 ou E3.

Ainsi, ces adénovirus défectifs ne peuvent être propagés que dans une lignée cellulaire complémentant *en trans* la fonction E1 essentielle à la réplication virale. A l'heure actuelle, la seule lignée cellulaire de complémentation utilisable est la lignée de rein embryonnaire 293 (Graham et al., 1977. J. Gen. Virol., *36*, 59-72), qui résulte de l'intégration dans ses chromosomes, don fragment du génome de l'Ad5 comprenant notamment l'extrémité 5' du génome viral ; de sorte que la lignée 293 complémente les adénovirus défectifs pour la fonction E1. Les cellules 293 contiennent des séquences qui se trouvent aussi dans l'adénovirus recombinant défectif, comme l'ITR 5', la région d'encapsidation et la partie en 3' de la région E1B comportant des séquences codant pour les protéines précoces.

La faisabilité du transfert de gènes en utilisant des adénovirus est maintenant établie. Mais, la question de leur innocuité reste posée. En effet, ils sont capables de transformer certaines lignées cellulaires en culture, ce qui reflète le pouvoir potentiellement oncogène de certains des produits d'expression du génome adénoviral, essentiellement de la région E1 et probablement E4, au moins pour certains sérotypes. De plus, la probabilité de recombinaison génétique entre un adénovirus défectif de l'art antérieur, notamment un adénovirus recombinant, et soit un adénovirus naturel ou sauvage (issu d'une contamination accidentelle ou d'une infection opportuniste d'un organisme hôte), soit un fragment de génome adénoviral intégré dans la lignée de complémentation 293 n'est pas négligeable. En effet, il suffit d'un événement de recombinaison pour restaurer la fonction E1 et générer un adénovirus recombinant non-défectif capable de se disséminer dans l'environnement. Il est aussi envisageable qu'un adénovirus naturel sauvage co-infectant la même cellule qu'un adénovirus défectif puisse complémenter ce dernier pour la fonction E1 provoquant une co-dissémination des deux virus. Enfin, certains types de cellules eucaryotes produisent des protéines présentant une activité E1 A-like également susceptibles de complémenter partiellement les adénovirus défectifs qui les infectent.

Il est donc souhaitable de disposer de vecteurs adénoviraux performants présentant le minimum de risque, en vue de leur utilisation en thérapie génique pour corriger *in vivo* des défauts génétiques graves et traiter certaines maladies pour lesquelles on ne dispose pas d'approches thérapeutiques efficaces. C'est de leur obtention que dépend le succès de la thérapie génique appliquée à l'homme.

De plus, il existe des interrogations à propos de l'obtention de la lignée 293. Ces interrogations peuvent être de nature à compromettre l'acceptabilité des produits destinés à un usage humain qui en seront dérivés. Il serait utile de disposer de lignées de complémentation dont l'origine et l'histoire sont exactement connues pour produire des particules d'adénovirus recombinants destinées à un usage humain.

On a maintenant trouvé (1) de nouveaux vecteurs adénoviraux défectifs délétés de certaines régions spécifiques du génome adénoviral et plus adaptés au transfert d'une séquence nucléotidique exogène *in vivo* et (2) de nouvelles lignées de complémentation caractérisées, acceptables d'un point de vue pharmaceutique et donc offrant toutes les caractéristiques de sécurité requises pour la production de produits destinés à un usage humain.

L'intérêt de ces nouveaux vecteurs est qu'ils présentent une capacité de clonage accrue permettant l'insertion d'un ou plusieurs gènes d'intérêt de grande taille et une sécurité d'emploi maximale. Ces mutations délétères rendent ces adénovirus incapables de réplication autonome et de transformation cellulaire et ceci sans altérer leur capacité à transférer et exprimer un gène d'intérêt.

Ces vecteurs adénoviraux sont défectifs pour la réplication, ils sont capables d'être encapsidé dans une cellule de complémentation, et ils sont dérivés du génome d'un adénovirus comprenant de 5' en 3', un ITR 5', une région d'encapsidation, une région E1A, une région E1B, une région E2, une région E3, une région E4 et un ITR 3', par délétion :
(i) de tout ou partie de la région E1A, et de l'intégralité de la partie de la région E18 codant pour les protéines précoces.

Au sens de la présente invention, l'expression "délétion" ou "dépourvu" se réfère à la suppression d'au moins un nucléotide dans la région ciblée et bien entendu il peut s'agir d'une délétion continue ou discontinue. Par tout ou partie, on entend soit l'intégralité soit une partie seulement de la région considérée. On préfère les délétions qui empêche la production d'au moins un produit d'expression codé par ladite région. Elles peuvent donc se situer dans une région codante ou dans une région régulatrice comme la région promotrice et concerner au moins un nucléotide de manière à détruire le cadre de lecture d'un gène ou rendre une région promotrice non-fonctionnelle. Il peut également s'agir de délétions partielles d'un ou plusieurs gènes de ladite région ou de l'ensemble de la région.

Un vecteur adénoviral comme décrit ci-dessus est défectif pour la replication mais capable d'être répliqué et encapsidé dans une cellule de complémentation lui fournissant *en trans* le ou les produit(s) pour lesquels il est défectif afin de générer une particule adénovirale (encore désignée adénovirus défectif) incapable de réplication autonome dans une cellule hôte mais néanmoins infectieuse car ayant la capacité de délivrer le vecteur dans une cellule hôte.

Selon une première variante, le vecteur adénoviral dérive du génome d'un adénovirus naturel ou sauvage par délétion de tout ou partie de la région E1A et de la partie de la région E1B comprenant l'intégralité des séquences codant pour les protéines précoces. Selon un mode préféré, elle concerne le promoteur et les séquences codant pour les produits d'expression de la région E1B c'est à dire les protéines précoces et n'inclut pas tout ou partie du signal de terminaison de la transcription qui recouvre les séquences codant pour la protéine tardive IX. S'agissant d'un vecteur adénoviral dérivant d'un adénovirus humain de type 5, ladite délétion comprend au moins les séquences comprises entre les nucléotides 1634 et 3509 du génome adénoviral dont la séquence est telle que divulguée dans la banque de donnée Genebank sous la référence M73260. Cette délétion a pour but de réduire ou supprimer les séquences communes entre un vecteur adénoviral comme décrit ci-dessus et le fragment de génome adénoviral intégré dans une lignée cellulaire de complémentation, par exemple la lignée 293. De plus, elle élimine d'un vecteur adénoviral selon l'invention des séquences dont les produits d'expression sont potentiellement oncogènes, du moins en conjonction avec les produits d'expression de la région E1A.

Par ailleurs, le vecteur adénoviral dérive en outre du génome d'un adénovirus naturel ou sauvage par délétion de tout ou partie :
- de la région E3.

Il va de soi que le vecteur adénoviral peut comporter une des trois délétions ci-dessus énoncées ou deux d'entre sites selon n'importe quelles combinaisons ou encore l'ensemble des délétions.

Selon un mode particulièrement avantageux, le vecteur adénoviral est délété d'une partie seulement de la région E3 et préférentiellement de la partie qui ne comprend pas les séquences codant pour la protéine gp 19kDa. La présence de la séquence codant pour la protéine gp19kDa dans le vecteur adénoviral, permettra aux cellules infectées d'échapper à l'immunosurveillance de l'hôte : un critère important lorsque le protocole thérapeutique nécessite plusieurs administrations répétées. On choisira, de préférence, de placer les séquences codant pour la gp19kDa sous le contrôle d'éléments appropriés permettant leur expression dans la cellule hôte, à savoir les éléments nécessaires à la transcription desdites séquences en ARNm et la traduction de ce dernier en protéine. Ces éléments comprennent en particulier un promoteur. De tels promoteurs sont bien connus de l'homme de l'art et sont insérés en amont de ladite séquence codante par les techniques conventionnelles du génie génétique. Le promoteur retenu sera, de préférence, un promoteur constitutif non activable par un des produits d'expression de la région E1A. A titre d'exemples, on peut citer le promoteur du gène HMG (Hydroxy-Methyl-Glutaryl coenzyme A réductase), le promoteur précoce du virus SV40 (Simian Virus 40), le LTR (Long Terminal Repeat) du RSV (Rous Sarcoma Virus) ou le promoteur d'un gène PGK (phospho-glycerate kinase) d'eucaryote supérieur.

Par ailleurs, le vecteur adénoviral, peut, de façon optionnelle, être délété de la partie de la région E3 correspondant à la région promotrice, laquelle sera substituée par une région promotrice hétérologue, telles l'une de celles mentionnées ci-dessus.

Comme précédemment, le vecteur adénoviral peut, en outre être dépourvu de tout ou partie des régions E1B et/ou E3 et, en particulier, selon un mode de réalisation tel que mentionné précédemment (comme la délétion de la partie de la région E1B comprenant l'intégralité des séquences codant pour les protéines précoces et de la partie de la région E3 ne codant pas pour la protéine gp19kDa).

Enfin selon une troisième variante, le vecteur adénoviral dérive du génome d'un adénovirus par délétion de tout ou partie de la région E1A et d'une partie de la région d'encapsidation.

Une délétion partielle de la région d'encapsidation permet de réduire notablement la probabilité de dissémination incontrôlée du vecteur adénoviral, lorsque ce dernier est en présence d'un adénovirus sauvage. Une telle délétion permet d'affecter ses fonctions d'encapsidation de telle sorte que même en cas de complémentation *en trans* de la fonction défective de celui-ci par un adénovirus sauvage, il ne pourra être encapsidé efficacement par rapport au génome de l'adénovirus sauvage compétiteur.

Les délétions de la région d'encapsidation sera choisies en fonction de 2 critères : une capacité réduite à être encapsidé mais simultanément une efficacité résiduelle compatible avec une production industrielle. En d'autres termes, la fonction d'encapsidation d'un vecteur adénoviral selon l'invention est substantiellement maintenue quoique à un degré moindre. L'atténuation peut être déterminée par les techniques conventionnelles de titrage par infection d'une lignée adéquate et évaluation du nombre de plages de lyse. De telles techniques sont connues de l'homme de l'art. Dans le cadre de l'invention, l'efficacité d'encapsidation est réduite d'un facteur 2 à 50, avantageusement 3 à 20 et de préférence 5 à 10 par rapport à un adénovirus témoin ayant une région d'encapsidation de type sauvage.

Bien entendu, le vecteur adénoviral atténué, peut en outre comprendre au moins une ou une quelconque combinaison des délétions précédemment citées.

Le vecteur adénoviral dérive du génome d'un adénovirus naturel ou sauvage, avantageusement d'un adénovirus canin, aviaire ou humain, de préférence d'un adénovirus humain de type 2, 3, 4, 5 ou 7 et, de manière tout à fait préférée, d'un adénovirus humain de type 5 (Ad5). Dans ce dernier cas, les délétions du vecteur adénoviral sont indiquées par référence à la position des nucléotides du génome de l'Ad5 spécifiée dans la banque de donnée Genebank sous la référence M73260.

On préfère tout particulièrement un vecteur adénoviral dérivant du génome d'un adénovirus humain de type 5, par délétion :
(i) de l'intégralité de la partie codant pour les protéines précoces de la région E1B et s'étendant du nucléotide 1634 et se terminant au nucléotide 4047 ; et/ou
(ii) de la partie de la région E3 s'étendant des nucléotides 27871 à 30748.

Dans le cadre de la présente invention, le vecteur adénoviral a pour objet le transfert et l'expression d'une séquence nucléotidique exogène dans une cellule hôte. Par "séquence nucléotidique exogène", on entend un acide nucléique qui comprend des séquences codantes et des séquences régulatrices permettant l'expression desdites séquences codantes et dans lequel les séquences codantes sont des séquences qui ne sont normalement pas présentes dans le génome d'un adénovirus. Les séquences régulatrices peuvent être d'origine quelconque. La séquence nucléotidique exogène est introduite dans le vecteur adénoviral par les techniques classiques du génie génétique, entre la région d'encapsidation et l'ITR 3'.

Une séquence nucléotidique exogène peut être constituée d'un ou plusieurs gène(s) d'intérêt et, de manière préférée, d'intérêt thérapeutique. Dans le cadre de la présente invention, un gène d'intérêt peut coder soit pour un ARN anti-sens, soit pour un ARNm qui sera ensuite traduit en protéine d'intérêt. Un gène d'intérêt peut être de type génomique, de type ADN complémentaire (ADNc) ou de type mixte (minigène, dans lequel au moins un intron est délété). Il peut coder pour une protéine mature, un précurseur d'une protéine mature, notamment un précurseur destiné à être secrété et comprenant de ce fait un peptide signal, une protéine chimérique provenant de la fusion de séquence d'origine diverse ou un mutant d'une protéine naturelle présentant des propriétés biologiques améliorées ou modifiées. Un tel mutant peut être obtenu par mutation, délétion, substitution et/ou addition d'un ou plusieurs nucléotide(s) du gène codant pour la protéine naturelle.

Un gène d'intérêt peut être placé sous le contrôle d'éléments appropriés à son expression dans une cellule hôte. Par "éléments appropriés", on entend l'ensemble des éléments nécessaires à sa transcription en ARN (ARN anti-sens ou ARNm) et à la traduction d'un ARNm en protéine. Parmi les éléments nécessaires à la transcription, le promoteur revêt une importance particulière. Il peut s'agir d'un promoteur constitutif ou d'un promoteur régulable et il peut être isolé d'un gène quelconque d'origine eucaryote ou virale et même adénovirale. Alternativement, il peut s'agir du promoteur naturel du gène d'intérêt en question. D'une façon générale, un promoteur en usage dans la présente invention, peut être modifié de manière à contenir des séquences régulatrices. A titre d'exemples, un gène d'intérêt en usage dans la présente invention, est placé sous le contrôle du promoteur des gènes d'immunoglobuline lorsque l'on cherche à cibler son transfert dans des cellules hôtes lymphocytaires. On peut également citer le promoteur du gène TK-HSV-1 (thymidine kinase du virus de l'herpès de type 1) ou encore le promoteur adénoviral MLP, notamment de l'adénovirus humain de type 2, permettant une expression dans un grand nombre de types cellulaires.

Parmi les gènes d'intérêt utilisables dans le cadre de la présente invention, on peut citer :
- les gènes codant pour des cytokines, comme l'interféron alpha, l'interféron gamma, les interleukines ;
- les gènes codant pour des récepteurs membranaires, comme les récepteurs reconnus par des organismes pathogènes (virus, bactéries, ou parasites), de préférence par le virus VIH (Virus de l'immunodéficience Humain) ;
- les gènes codant pour des facteurs de coagulation, comme le facteur VIII et le facteur IX ;
- le gène codant pour la dystrophine ;
- le gène codant pour l'insuline ;
- les gènes codant pour des protéines participant directement ou indirectement aux canaux ioniques cellulaires, comme la protéine CFTR (Cystic Fibrosis Transmembrane Conductance Regulator) ;
- les gènes codant pour des ARN anti-sens ou des protéines capables d'inhiber l'activité d'une protéine produite par un gène pathogène, présent dans le génome d'un organisme pathogène, ou par un gène cellulaire, dont l'expression est dérégulée, par exemple un oncogène ;
- les gènes codant pour une protéine inhibant une activité enzymatique, comme l'α1- antitrypsine ou un inhibiteur d'une protéase virale ;
- les gènes codant pour des variants de protéines pathogènes qui ont été mutées de façon à altérer leur fonction biologique, comme par exemple des variants trans-dominants de la protéine TAT du virus VIH capables de compétition avec la protéine naturelle pour la liaison à la séquence cible, empêchant ainsi l'activation du VIH;
- les gènes codant pour des épitopes antigéniques afin d'accroître l'immunité de la cellule hôte;
- les gènes codant pour les protéines de complexe majeur d'histocompatibilité des classes I et II, ainsi que les gènes codant pour les protéines inductrices de ces gènes ;
- les gènes codant pour des enzymes cellulaires ou produites par des organismes pathogènes ; et
- les gènes suicides. On peut citer plus particulièrement le gène suicide TK-HSV-1. L'enzyme TK virale présente une affinité nettement supérieure par rapport à l'enzyme TK cellulaire pour certains analogues de nucléosides (comme l'acyclovir ou le gancyclovir). Elle les convertit en molécules monophosphatées, elles-mêmes convertibles, par les enzymes cellulaires, en précurseurs de nucléotides, qui sont toxiques. Ces analogues de nucléotides sont incorporables dans les molécules d'ADN en voie de synthèse, donc principalement dans l'ADN des cellules en état de réplication. Cette incorporation permet de détruire spécifiquement les cellules en division comme les cellules cancéreuses.

Cette liste n'est pas limitative et d'autres gènes d'intérêt peuvent être utilisés dans le cadre de la présente invention.

Par ailleurs, selon un autre mode de mise en oeuvre de l'invention, le vecteur adénoviral peut en outre comprendre un gène non-thérapeutique codant pour une protéine trans-activatrice de la transcription non-adénovirale. Bien-entendu, on évitera le ou les gênes(s) de la région E1A codant pour une protéine trans-activatrice, dont l'expression risquerait de rendre l'adénovirus non-défectif. On choisira, de préférence, le gène codant pour la protéine Gal4 de *Saccharomyces cerevisiae.* Son expression permettra la propagation du vecteur dans une lignée de complémentation telle que celle décrite ci-après. Une telle lignée est plus sophistiquée et permet de pallier à d'éventuels problèmes de toxicité due à la production en continue des protéines adénovirales de complémentation. Le gène codant pour une protéine trans-activatrice de la transcription peut être placé, si nécessaire, sous le contrôle d'éléments appropriés à son expression ; par exemple ceux qui permettent l'expression d'un gène d'intérêt.

Le vecteur adénoviral peut être compris dans une cellule eucaryote hôte sous forme d'un vecteur adénoviral ou sous forme d'une particule adénovirale. Ladite cellule est avantageusement une cellule de mammifère et, de préférence, une cellule humaine et peut comprendre ledit vecteur sous forme intégrée dans le génome ou, de préférence, sous forme non-intégrée (épisome).

La particule adénovirale peut être préparée par passage dans toute lignée cellulaire de complémentation fournissant *en trans* les fonctions pour lesquelles le vecteur adénoviral comme décrit ci-dessus est défectif, par exemple la lignée 293 de l'art antérieur. Ces techniques de préparation sont connues de l'homme de l'art (Graham et Preveo, 1991, Methods in Molecular Biology, vol 7, 109-128, Ed : E.J. Murey, The Human Press Inc.). D'une manière optionnelle, la particule adénovirale peut être générée dans une lignée cellulaire de complémentation selon l'invention telle que décrite ci-après.

C'est pourquoi la présente invention concerne une lignée de complémentation propre à la production de particules d'adénovirus destinées à un usage thérapeutique ou prophylactique humain ladite lignée cellulaire de complémentation comportant un élément de complémentation; ledit élément de complémentation étant caractérisé en ce qu'il comprend comme seul ADN adénoviral la partie de la région E1A codant pour les protéines précoces de la région E1A et l'intégralité des séquences codant pour les protéines précoces de la région E1B placée sous le contrôle de son propre promoteur et en ce que l'expression de la partie de la région E1A est placée le contrôle d'un promoteur hétérologue; et ledit élément de complémentation
(a) étant capable de complémenter en trans un vecteur adénoviral qui est dérivé d'un adénovirus humain et qui est défectif pour la fonction E1; et
(b) étant intégré dans le génome de ladite lignée cellulaire de complémentation.

Dans le cadre de la présente invention, le terme "lignée cellulaire de complémentation" se réfère à une cellule eucaryote capable de fournir en trans la ou les fonction(s) pour la(les)quelle(s) un vecteur adénoviral est défectif. En d'autres termes, elle est capable de produire les protéines précoces de la région E1A et les protéines précoces de la région E1B nécessaire(s) à la replication dudit vecteur adénoviral, protéines précoces qu'il ne peut lui même produire et qui sont nécessaires à la constitution d'une particule virale. Ainsi, un vecteur adénoviral humain défectif pour la fonction E1 devra être propagé dans une lignée cellulaire de complémentation pour E1 (capable de fournir en trans la ou l'ensemble des protéines codées par la région E1 que le vecteur ne peut produire). Comme indiqué dans l'introduction, la région E3 est non essentielle, et ne nécessite pas d'être spécifiquement complétée.

Une lignée cellulaire de complémentation selon l'invention peut être dérivée soit d'une lignée cellulaire immortalisée, capable de se diviser indéfiniment, soit d'une lignée primaire. Conformément aux buts poursuivis par la présente invention, une lignée cellulaire de complémentation selon l'invention est utile pour l'encapsidation de n'importe quel vecteur adénoviral défectif et, en particulier, d'un vecteur adénoviral défectif comme décrit ci-dessus. Ainsi, lorsqu'on utilisera ci-après le terme "vecteur adénoviral défectif", il doit être entendu qu'il fait référence à un vecteur défectif quelconque, de l'art antérieur ou comme décrit ci-dessus.

L' "élément de complémentation" est intégré dans le génome d'une lignée cellulaire de complémentation, selon l'invention. Les méthodes pour introduire un vecteur ou un acide nucléique dans une lignée cellulaire et éventuellement l'intégrer dans le génome d'une cellule constituent des techniques conventionnelles bien connues de l'homme de l'art, de même que les vecteurs utilisables à de telles fins. L'élément de complémentation peut être introduit dans une lignée de complémentation selon l'invention, de façon préalable ou concomitante à un vecteur adénoviral défectif.

Une lignée cellulaire de complémentation selon l'invention est destinée à complémenter en trans un vecteur adénoviral qui est dérivé d'un adénovirus humain et qui est défectif pour la fonction E1. Une telle lignée présente l'avantage de diminuer les risques de recombinaison puisque, contrairement à la lignée conventionnelle 293, elle est dépourvue de l'ITR 5' présent dans les vecteurs.

Une lignée cellulaire de complémentation selon l'invention est dépourvue de la région E1A. La partie du génome adénoviral codant pour les protéines précoces de ladite région E1A est placée sous le contrôle d'un promoteur hétérologue approprié et fonctionnel dans ladite lignée cellulaire de complémentation. Il peut être isolé de n'importe quel gène eucaryote ou viral. On évitera, cependant, d'avoir recours à un promoteur adénoviral d'une région précoce. Il peut s'agir d'un promoteur constitutif. A titre d'exemples, on peut citer les promoteurs du virus SV40, du gène TK-HSV-1 et du gène murin PGK.

D'une manière alternative, le promoteur retenu peut être régulable et avantageusement, inductible par une protéine trans-activatrice de transcription non adénovirale. Il peut s'agir d'un promoteur isolé d'un gène naturellement inductible ou d'un promoteur quelconque modifié par l'addition de séquences d'activation (ou UAS, pour Upstream Activating Sequence en anglais) répondant à ladite protéine trans-activatrice. De manière plus particulière, on préfére utiliser un promoteur inductible par la protéine Gal4 de *Saccharomyces cerevisiae* et, de préférence, un promoteur hybride constitué d'un promoteur dit "minimum" contenant uniquement les séquences d'initiation de la transcription (TATA box et site d'initiation) d'un gène quelconque (par exemple du gène TK-HSV-1, en amont duquel on a inséré au moins une séquence d'activation du gène Gal10 de *Saccharomyces cerevisiae* (Webster et al., 1988, Cell, *52*, 169-178). Cette dernière peut être synthétisée chimiquement ou isolée du gène Gal10, selon les techniques classiques du génie génétique. Ainsi, le promoteur hybride ne sera activé et n'induira l'expression des gènes codés par la région E1A placés sous son contrôle, qu'en présence de la protéine Gal4.
Ainsi, l'induction peut être déclenchée en présence d'un vecteur adénoviral défectif comme décrit ci-dessus exprimant la protéine Gal4. Cependant une telle lignée peut également être utilisée pour préparer n'importe quel vecteur adénoviral défectif, à la condition toutefois de fournir *en trans* la protéine Gal4. Les moyens de fournir *en trans* une protéine sont connus de l'homme du métier.

Une lignée cellulaire de complémentation comprend une partie du génome d'un adénovirus humain qui, dérive avantageusement d'un adénovirus humain du type 2 ou 5.

Selon un mode préféré, une lignée cellulaire de complémentation selon l'invention peut comporter un élément de complémentation comprenant, en outre, un gène codant pour un marqueur de sélection permettant la détection et l'isolement des cellules le comportant. Dans le contexte de la présente invention, il peut s'agir de n'importe quel gène codant pour un marqueur de sélection, ceux-ci étant généralement connus de l'homme de l'art, avantageusement d'un gène de résistance à un antibiotique et, de préférence, du gène codant pour la puromycine acetyl-transférase (gène pac) conférant la résistance à la puromycine.

Dans le cadre de la présente invention, le gène codant pour un marqueur de sélection peut être placé sous le contrôle des éléments appropriés permettant son expression. Il peut s'agir d'un promoteur constitutif, comme le promoteur précoce du virus SV40.
Une telle combinaison introduira une pression de sélection pour maintenir l'expression, des gènes de la région E1A dans une lignée cellulaire de complémentation selon l'invention. Aux fins de la présente invention, le promoteur retenu peut être modifié par délétion, mutation, substitution et/ou addition de nucléotides.

Selon un mode de réalisation tout à fait préféré, une lignée cellulaire de complémentation selon l'invention est dérivée d'une lignée cellulaire acceptable d'un point de vue pharmaceutique. Par "lignée cellulaire acceptable d'un point de vue pharmaceutique", on entend une lignée cellulaire caractérisée (dont on connait l'origine et l'histoire) et/ou ayant déjà été utilisée pour la production à grande échelle de produits destinés à un usage humain (constitution de lots pour des essais cliniques avancés ou de lots destinés à la vente). De telles lignées sont disponibles dans des organismes tels que l'ATCC. A cet égard, on peut mentionner les lignées de rein de singe vert d'Afrique Vero, humaine dérivée d'un carcinome de poumon A549, humaine pulmonaire MRC5 et humaine pulmonaire W138.

D'une manière alternative, une lignée cellulaire de complémentation selon l'invention peut dériver de cellules primaires et notamment de cellules de rétine prélevées d'un embryon humain.

L'invention a également pour object l'usage d'une lignée cellulaire de complémentation selon l'invention pour la préparation d'une composition pharmaceutic pour transférer des molécules d'ADN dans une cellule ou un organisme eucaryote.

La présente invention est enfin relative à une composition pharmaceutique comprenant à titre d'agent thérapeutique ou prophylactique une cellule de complémentation selon l'invention, en association avec un support acceptable d'un point de vue pharmaceutique.

La composition selon l'invention, est en particulier destinée au traitement préventif ou curatif de maladies telles que :
- des maladies génétiques, comme l'hémophilie, la mucoviscidose ou la myopathie, celle de Duchêne et de Becker,
- des cancers, comme ceux induits par des oncogènes ou des virus,
- des maladies rétrovirales, comme le SIDA (syndrome de l'immunodéficience acquise résultant de l'infection par le VIH), et
- des maladies virales récurrentes, comme les infections virales provoquées par le virus de l'herpès.

Une composition pharmaceutique selon l'invention peut être fabriquée de manière conventionnelle. En particulier, on associe une quantité thérapeutiquement efficace d'un agent thérapeutique ou prophylactique à un support tel qu'un diluant. Une composition selon l'invention peut être administrée par aérosol ou par n'importe quelle voie conventionnelle en usage dans le domaine de l'art, en particulier par voie orale, sous-cutanée, intramusculaire, intraveineuse, intrapéritonéale intrapulmonaire ou intra-trachéale. L'administration peut avoir lieu en dose unique ou répétée une ou plusieurs fois après un certain délai d'intervalle. La voie d'administration et le dosage appropriés varient en fonction de divers paramètres, par exemple, de l'individu traité ou de la maladie à traiter ou encore du ou des gène (s) d'intérêt à transférer. D'une manière générale, une composition pharmaceutique selon l'invention comprend une dose d'adénovirus selon l'invention comprise entre 10⁴ et 10¹⁴, avantageusement 10⁵ et 10¹⁹ et de préférence 10⁶ et 10¹¹. Une composition pharmaceutique, en particulier à visée prophylactique, peut comprendre en outre un adjuvant acceptable d'un point de vue pharmaceutique.

La Figure 1 est une représentation schématique du génome de l'adénovirus humain de type 5 (représenté en unités arbitraires de 0 à 100), indiquant l'emplacement des différents gènes.

La Figure 2 est une représentation schématique du vecteur pTG6548.

La Figure 3 est une représentation schématique du vecteur pTG6581.

La Figure 4 est une représentation schématique du vecteur pTG6303.

La Figure 5 est une représentation schématique des vecteurs pTG1660 et pTG1661.

La Figure 6 est une représentation schématique des vecteurs pTG1653, pTG1654 et pTG1655.

La Figure 7 est une représentation schématique du vecteur pTG5913.

La Figure 8 est une représentation schématique du vecteur pTG8512

La Figure 9 est une représentation schématique du vecteur pTG8513.

La Figure 10 est une représentation schématique du vecteur pTG8514.

La Figure 11 est une représentation schématique du vecteur pTG8515.

### EXEMPLES DE REFERENCE

Les exemples suivants n'illustrant pas l'invention telle que revendiquée.

Les constructions décrites ci-dessous sont réalisées selon les techniques générales de génie génétique et de clonage moléculaire, détaillées dans Maniatis et al., (1989, Laboratory Manual, Cold Spring Harbor, Laboratory Press, Cold Spring Harbor, NY). L'ensemble des étapes de clonage mettant en oeuvre des plasmides bactériens est réalisé par passage dans la souche *Escherichia coli (E. coli*) 5K ou BJ, alors que celles qui mettent en oeuvre des vecteurs dérivés du phage M13 sont réalisées par passage dans *E. coli* NM 522. En ce qui concerne les étapes d'amplification par PCR, on applique le protocole tel que décrit dans PCR Protocols-A guide to methods and applications, (1990, edité par Innis, Gelfand, Sninsky and White, Académic Press Inc.).

D'autre part, les cellules sont transfectées selon les techniques standards bien connues de l'homme du métier. On peut citer la technique au phosphate de calcium (Maniatis et al., *supra).* Mais d'autres protocoles permettant d'introduire un acide nucléique dans une cellule peuvent également être employés, tels que la technique au DEAE dextran, l'électroporation, les méthodes basées sur les chocs osmotiques, la microinjection d'une cellule sélectionnée ou les méthodes basées sur l'emploi de liposomes.

Les fragments insérés dans les différentes constructions décrites ci-après, sont indiqués précisément selon leur position dans la séquence nucléotidique :
- du génome de l'Ad5 telle que divulguée dans la banque de données Genebank sous la référence M7 3260,
- du génome de l'adénovirus de type 2 (Ad2), telle que divulguée dans la banque de données Genebank sous la référence J01949.
- du génome du virus SV40 telle que divulguée dans la banque de données Genebank sous la référence J02400.

### EXEMPLE 1 : Génération d'un adénovirus "atténué" comprenant une délètion d'une partie de la région d'encapsidation.

### 1. Construction d'un vecteur "atténué" comprenant une délétion au nucléotide 184 au nucléotide 273 de la région d"encapsidation

On construit un vecteur comprenant
- l'ITR 5' du génome de l'Ad5 (du nucléotide 1 au nucléotide 103),
- la région d'encapsidation de l'Ad5 comprise entre les nucléotides 104 à 458 dans laquelle la portion allant du nucléotide 184 au nucléotide 273 est délétée et la thymine (T) en position 176 est modifiée en une cytosine (C) afin de créer un site de restriction *Aat*ll,
- une cassette d'expression d'un gène d'intérêt comprenant de 5' vers 3' le MLP de l'Ad2 (nucléotides 5779 à 6038), les sites de restriction *Kpn*l*-Xba*l*-Hind*lll et *Bam*HI, l'ADNc humain codant pour la protéine CFTR, (la composition en acides aminés correspond à la séquence publiée par Riordan et al, 1989, Science, *245*, 1066-1073 ; à l'exception d'une valine à la place de la méthionine en position 470), les sites *Pst*l, *Xho*l et *Sal*l et enfin le signal de terminaison de la transcription du virus SV40 (nucléotides 2665 à 2538), et
- le fragment du génome de l'Ad5 s'étendant du nucléotide 3329 au nucléotide 6241.

Dans un premier temps, on clone entre les sites *Eco*RI et *Eco*RV du vecteur M13TG131 (Kieny et al., 1983, Gene, *26*, 91-99) le fragment *Eco*RI *Sma*l isolé de pMLP11. Cette construction est issue de pMLP10 (Levrero et al., 1991, Gene, *101*,195-202) et diffère du vecteur parental par l'introduction d'un site *Sma*l au niveau du site *Hind*lll. On obtient le vecteur M13TG6501. Celui-ci est soumis à une mutagénèse dirigée afin de déléter les séquences comprises entre les nucléotides 184 à 273 de la région d'encapsidation. La mutagénèse dirigée est réalisée à l'aide d'un kit commercial (Amersham) selon les recommandations du fournisseur, et met en oeuvre l'oligonucléotide OTG4174 reporté dans l'identificateur de séquence n°1 (SEQ ID NO: 1). Le vecteur muté est désigné M13TG6502. La région d'encapsidation ainsi délétée est réintroduite sous forme d'un fragment *Eco*RI*-Bgl*ll, le site *Bgl*ll étant rendu franc par traitement à l'ADN polymérase Klenow, dans le vecteur pMLP11 digéré par *Eco*RI et *Sma*l.

Le vecteur obtenu, pTG6500, est digéré partiellement par *Psl*l, traité à l'ADN polymérase du phage T4 puis digéré par *Pvu*l. On insère dans ce vecteur le fragment *Pvu*l*-Hpa*l isolé de pTG5955 (dérivé de pMLP11). Ce fragment comporte la signal de terminaison de la transcription du virus SV40 et la partie du génome de l'Ad5 s'étendant du nucléotide 3329 au nucléotide 6241. Le vecteur pTG6505 ainsi généré est digéré partiellement par *Sph*l, traité à l'ADN polymérase du phage T4 et religué, ceci afin de détruire le site *Sph*l situé en 5' du polylinker. Il résulte le pTG6511, dans lequel on clone, après digestion par *Bam*HI et traitement à l'ADN polymérase Klenow, l'ADNc CFTR humain sous forme d'un fragment aux extrémités franches généré par digestion *Xho*l et *Ava*l et traitement à l'ADN polymérase Klenow. On obtient pTG6525. A titre indicatif, l'ADNc CFTR est isolé d'un plasmide de l'art antérieur, tel que pTG5960 (Dalemans et al., 1991, Nature, *354*, 526-528).

### 2. Construction d'un vecteur "atténué" comprenant une délétion au nucléotide 270 au nucléotide 346 de la région d'encapsidation.

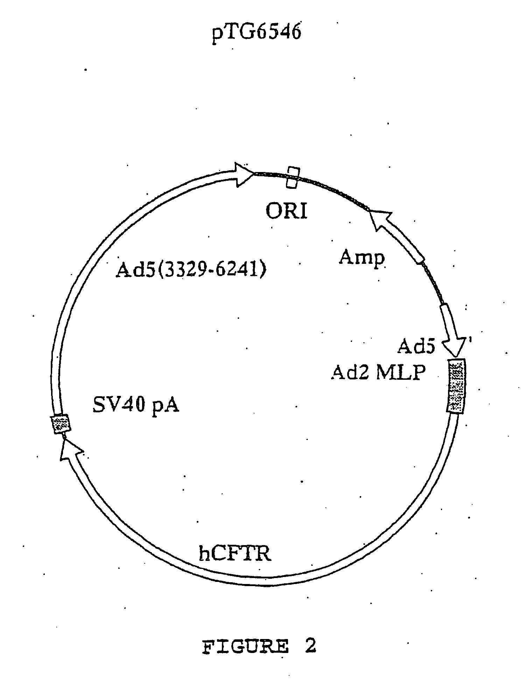

Le vecteur M13TG6501 est soumis à une mutagénèse dirigée mettant en oeuvre l'oligonucléotide OTG4173 (SEQ ID NO: 2). Puis le fragment muté est réintroduit dans pMLP11, comme indiqué précédemment, pour générer la vecteur pTG6501. Ce dernier est digéré par S*ph*l, traité à l'ADN polymérase du phage T4, puis par *Pvu*l*.* On obtient pTG6546 (Figure 2) par clonage du fragment *Pvu*l-*Kpn*l (le site *Kpn*l ayant été rendu franc) isolé de pTG6525 et comportant l'ADNc CFTR humain.

### 3. Construction d'un vecteur "atténué" comprenant une délétion du nucléotide 287 au nucléotide 358 de la région d'encapsidation.

Le vecteur M13TG6501 est soumis à une mutagénèse dirigée afin de déléter les séquences comprises entre les nucléotides 287 et 358 de la région d'encapsidation et de modifier les thymines en position 275 et 276 en guanines pour introduire un site *Nco*l*.* La mutagénèse est réalisée à l'aide de l'oligonucléotide OTG4191 (SEQ ID NO: 3) pour donner M13TG6507. Ce dernier est clivé par *Bgl*ll, traité à l'ADN polymérase Klenow puis digéré par *Eco*RI et on purifie le fragment correspondant muté que l'on introduit dans pMLP11 digéré par *Ec*oRI et *Sma*l. On génère pTG6504, duquel on isole le fragment *Sph*l (site rendu franc par traitement à l'ADN polymérase du phage T4)-*Pvu*l que l'on insère entre les sites *Kpn*l (rendu franc par traitement à la T4 polymérase) et *Pvu*l de pTG6511. On obtient pTG6513 qui est traité par *Bam*HI et l'ADN polymérase Klenow avant d'insérer le fragment *Ava*l et *Xho*l de pTG5960 pour donner pTG6526.

### 4. Génération d'un adénovirus recombinant défectif et atténué.

Les adénovirus défectifs recombinants sont générés par co-transfection dans les cellules 293 de soit pTG6525, pTG6526 ou pTG6546 linéarisé par *Cla*l et d'ADN génomique de l'Ad-dl324 (Thimmappaya et al., 1982, Cell, *31*, 543-551) également digéré par *Cla*l*,* de manière à générer un virus recombinant par recombinaison homologue. Après 8 à 10 jours, les plages individuelles sont isolées, amplifiées dans les cellules 293 et analysées par cartographie de restriction. Des stocks viraux (AdTG6525, AdTG6526 et AdTG6546) sont constitués et leur titre déterminé selon les techniques conventionnelles.

Le virus AdTG6546 est placé en situation de compétition par co-infection avec l'Ad-CFTR (Rosenfeld et al., 1992, Cell, *68*, 143-155) qui comporte une région d'encapsidation de type sauvage. On infecte les cellules 293 par 5 ufp (unité formant des plages) d'Ad-CFTR et 5 ufp d'AdTG6546 par cellule. On isole en parallèle l'ADN viral total par la méthode de Hirt (Gluzman et Van Doren, 1983, J. Virol., *45*, 91-103) et l'ADN viral encapsidé après traitement des cellules avec 0,2 % déoxydrolate puis avec 10 µg/ml de déoxyribonucléase (DNase) I, pour éliminer les ADN non protégés dans les virions. Alors que la quantité d'ADN total d'Ad-CFTR et d'AdTG6546 est identique, il y a environ 3 fois moins d'ADN d'AdTG6546 que d'ADN d'Ad-CFTR encapsidé.

On mesure le niveau d'expression de la protéine CFTR dans les extraits cellulaires de cellules 293 infectées par AdTG6546. L'analyse est effectuée par Western blot selon la technique décrite dans Dalemans et al. (1991, Nature, *supra)* en mettant en oeuvre l'anticorps monoclonal MATG1031. Mais, tout autre anticorps reconnaissant des épitopes antigéniques de la protéine CFTR peut être utilisé. On révèle un produit d'une masse moléculaire attendue d'environ 170 kDa. A titre indicatif, le niveau de production est à peu près équivalent à celui obtenu dans les extraits cellulaires infectés par le virus non atténué Ad-CFTR.

### EXEMPLE 2 : Génération d'un adénovirus défectif délété de la région E1A et de l'intégralité des séquences codant pour les protéines précoces de la région E1B.

### 1. Obtention d'un adénovirus recombinant pour l'expression de la protéine CFTR (AdTG6581)

Un tel adénovirus est généré à partir d'un vecteur plasmidique pTG6581 comprenant de 5' vers 3':
- l'ITR 5' de l'Ad5 (des nucléotides là 103),
- la région d'encapsidation de l'Ad5 (des nucléotides 104 à 458),
- une séquence nucléotidique exogène comportant une cassette d'expression, laquelle comprend les éléments suivants :
   * le MLP de l'Ad2 (nucléotides 5779 à 6038), suivi des trois leaders tripartites également de l'Ad2 (nucléotides 6039-6079 ; nucléotides 7101-7175 ; nucléotides 9637-9712); ces leaders sont inclus afin d'augmenter l'efficacité de traduction des séquences insérées en aval,
   * un polylinker comprenant de 5' vers 3' les sites de restrictions *Xha*l, *Hind*lll, *Bam*HI *Eco*RV, *Hpa*l et *Not*l utilisables pour le clonage d'un gène d'intérêt,
   * un gène d'intérêt, comme le gène codant pour la protéine CFTR,
   * le signal de terminaison de la transcription isolé du virus SV40 (nucléotides 2543 à 2618),
- la portion du génome adénoviral de l'Ad5 allant des nucléotides 4047 à 6241.

Le fragment du génome de l'Ad5 s'étendant du nucléotide 4047 au nucléotide 4614 est amplifié par PCR à partir de l'ADN génomique d'Ad5. La réaction PCR met en oeuvre l'amorce sens OTG5021 (SEQ ID NO:4), comprenant en son extrémité 5' un site *Bam*HI destiné à faciliter les étapes de clonage ultérieures, et l'amorce anti-sens OTG5157 (SEQ ID NO: 5.) Le fragment ainsi généré est traité à l'ADN polymérase Klenow, avant d'être cloné dans le site *Sma*l de M13mp18 (Gibco BRL), donnant lieu au M13G6517. La séquence du fragment généré par PCR est vérifiée selon la méthode enzymatique classique (Sanger et al., 1977, Proc. Natl. Acad. Sci. USA, *74*, 5463).

Par ailleurs, le fragment *Pvu*l-*Sma*l est isolé de pMLP11. Il est cloné entre les sites *Pvu*l et *Kpn*l de pTG6511 (exemple 1.1 ), le site *Kpn*l ayant été rendu franc par un traitement à l'ADN polymérase du phage T4 selon les méthodes standards. On génère ainsi la vecteur pTG6547.

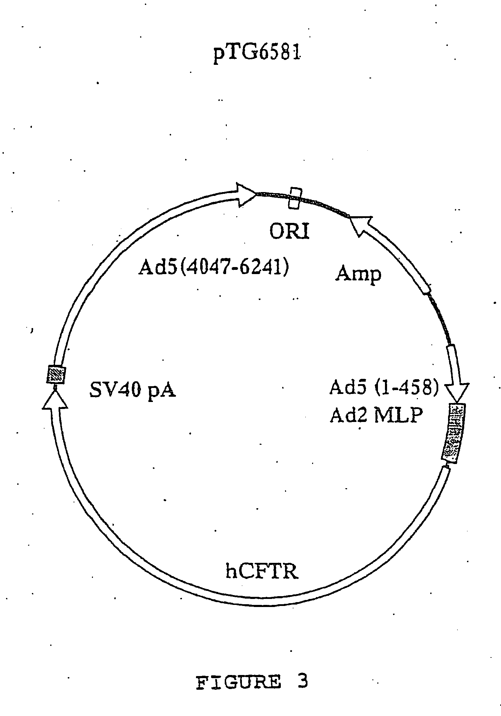

Ce dernier est digéré par les enzymes *Sal*l et *Bst*XI et ligué à deux fragments, d'une part le fragment *Bam*HI-*Bst*XI purifié de M13TG6517 et, d'autre part, le fragment *Xho*l-*Bgl*l de pTG6185. Ce dernier comprend notamment le signal de terminaison de la transcription du virus SV40 encadré par les sites de restriction *Xho*I et *Bgl*ll*.* Mais, tout autre plasmide comportant la même séquence de terminaison et des sites de restriction adéquates pourrait être utilisé. On obtient le vecteur pTG6555, dans lequel on insère dans le site unique *Bam*Hl un adaptateur contenant deux sites de restriction générant des extrémités franches, *Eco*RV et *Hpa*l. Cet adaptateur provient de la réassociation des oligonucléotides OTG5564 et OTG5565 (SEQ ID NO: 6 et 7). On obtient pTG6580. Enfin, le fragment *Sac*l-*Pst*l de pTG6525 dont les extrémités ont été rendues franches et comportant l'ADNc CFTR humain, est cloné dans le site *Eco*RV de pTG6580. On génère pTG6581 (Figure 3).

L'adénovirus recombinant correspondant AdTG6581 est généré par co-transfection de pTG6581 et Ad d1324 clivés par *Cla*l dans une lignée de complémentation pour la fonction E1, comme la lignée 293 ou une lignée de l'exemple 6, selon la protocole classique.

### 2 Obtention d'un adénovirus recombinant our l'expression de l'IFNγ.

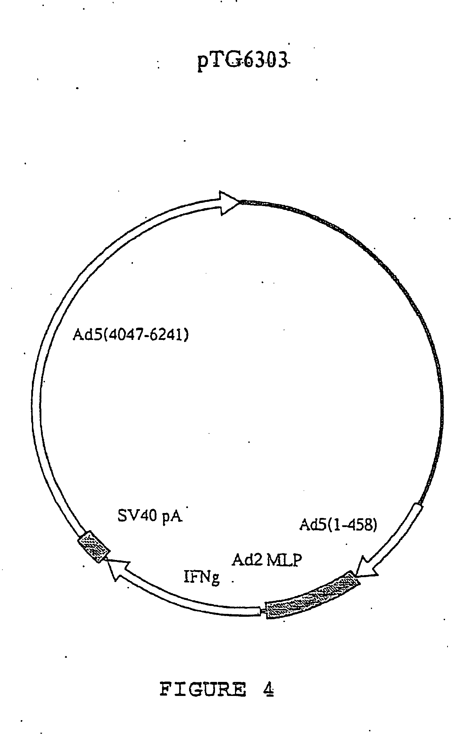

Le vecteur pTG6303 (Figure 4) est obtenu par clonage dans le site *Hpa*l de pTG6580 du fragment *Hpa*l-*Sma*l de M13TG2437. Ce dernier provient du clonage dans un vecteur M13TG130 (Kleny et al., 1983, *supra*) du gène codant pour l'interféron gamma (IFNγ) dont la séquence est telle que spécifiée dans Gray et al. (1982, Nature, *295*, 503-508). L'adénovirus recombinant AdTG6303 est obtenu selon les techniques classiques par recombinaison homologue résultant de la co-transfection de pTG6303 et de l'Ad d1324 linéarisé par *Cla*l dans une lignée de complémentation pour la fonction E1.

### 3. Construction d'un adénovirus délété de la région E1 et dans lequel la région E3 est placée sous le contrôle d'un promoteur constitutif.

Le vecteur pTG1670 est obtenu par clonage entre les sites *Aat*ll et *Bam*HI du vecteur p polyll (Lathe et al., 1987, Gene *57*, 193-201), d'un fragment PCR comportant le LTR3' (Long Terminal Repeat) du virus RSV (Rous Sarcoma Virus). La réaction PCR met en oeuvre le vecteur pRSV/L (De Wet et al., 1987, Mol. Cell. Biol. *7*, 725-737) à titre de matrice et les amorces OTG5892 et OTG5893 (SEQ ID NO: 8 et 9).

Par ailleurs, la partie 5' de la région E3 (nucléotides 27588 à 28607) est amplifiée par PCR à partir du vecteur pTG1659 et à l'aide des amorces OTG5920 et OTG5891 (SEQ ID NO: 10 et 11). Ce dernier est construit en plusieurs étapes. Le fragment *Bam*Hl*-Avr*ll (nucléotides 21562 à 28752) est obtenu de l'ADN génomique d'Ad5 puis cloné entre les mêmes sites de pTG7457 pour générer pTG1649. Le vecteur pTG7457 est un pUC19 (Gibco BRL) modifié au niveau du polylinker de manière à contenir notamment un site *Avr*ll*.* Puis on introduit le fragment *Eco*RI (Klenow)-*Avr*ll de M13TG1646 (exemple 8) dans pTG1649 clivé par *Avr*ll*-Nde*l (Klenow), ce qui donne le vecteur pTG1651. Enfin, pTG1659 est généré par l'insertion du fragment *Avr*ll (nucléotides 28752 à 35463) purifié de l'ADN génomique d'Ad5 dans pTG1651 linéarisé par *Avr*ll. Le fragment PCR est intégré entre les sites *Xba*l et *Bam*HI et de p poly II, pour donner pTG1671. On insère ensuite dans le site *Aat*ll de ce dernier, un fragment *Eco*RV*-Aat*ll obtenu de pTG1670, pour donner pTG1676.

Le fragment *Eco*RI de l'Ad5 correspondant aux nucléotides 27331 à 30049, est isolé à partir d'une préparation d'ADN génomique et sous-cloné dans le pBluescript-Sk+ (Stratagène) préalablement clivé par *Eco*RI. On obtient pTG1669. Celui-ci est muté (kit Amersham) par introduction d'un site *Bam*Hl soit en position 27867 (oligonucléotide mutagène OTG6079 : SEQ ID NO:12) ou en position 28249 (oligonucléotide mutagène OTG6080; SEQ ID NO: 13). On obtient respectivement pTG1672 et pTG1673. On isole du vecteur pTG1676, le fragment *Bam*HI-*Bsi*WI comportant le LTR 3' de RSV suivi de la partie 5' de la région E3 et on l'insère entre les sites *Bam*HI (position 27331 ou 30049) et *BsiW* (position 28390) des vecteurs obtenus à l'étape précédente pour générer pTG1977 et pTG1978. Puis le fragment *Eco*RI obtenu de chacun de ces deux vecteurs est intégré dans pTG1679, en remplacement du fragment *Eco*RI sauvage. On obtient pTG1679-E3+. A titre indicatif, le vecteur pTG1679 résulte du clonage du fragment *Bst*EII-*Kpn*l (site rendu franc par traitement à la T4 polymerase) de pTG6590 (exemple 3.1) entre les sites *Bst*EII*-Bam*HI (site rendu franc par traitement à la Klenow polymérase) de pTG65B4 (exemple 3.1).

On génère une particule d'adénovirus par recombinaison homologue dans une lignée de complémentation pour la fonction E1, entre le fragment *Aat*ll de pTG 1679-E3+ et un vecteur adénoviral tel que l'Ad dl324 ou Ad-RSVβ-gal. Ce dernier contient le gène de la β-galactosidase à la place de la région E1 (Stratford-Perricaudet et al., 1992, J. Clin. Invest., *90*, 626-630).

### EXEMPLE 3: Construction d'un vecteur adénoviral recombinant à capacité de clonage améliorée par délétion partielle des régions E1 et E3

### 1. Construction de pTG6590△E3

Le fragment portant la partie du génome de l'Ad5 comprise entre les nucléotides 27325 et 27871; est amplifié par PCR à partir d'une préparation d'ADN génomique d'Ad5 et à l'aide des amorces OTG6064 et OTG6065 (SEQ ID NO: 14 et 15). OTG6065 comprend à son extrémité 5' un site *Bsm*I, également présent dans la région E3 (en position 30750).

Le fragment amplifié est cloné dans le site *Sma*l de M13mp18, pour donner M13TG6523. Le fragment *Eco*RI-*Bsm*l est isolé de ce dernier pour être introduit dans le vecteur pTG6590 clivé par les mêmes enzymes. On obtient pTG6590Δ3, lequel contient la partie 3' du génome adénoviral (des nucléotides 27082 à 35935) délétée de la région E3 comprise entre les nucléotides 27872 à 30740, alors que pTG6590 est délété d'une partie plus petite de la région E3 (position 28592 à 30470). Le vecteur pTG6590 est obtenu de la façon suivante: on génère par PCR un fragment s'étendant des nucléotides 35228 à 35935 (comportant l'ITR 3') à partir d'une préparation génomique d'Ad5 et à l'aide des amorces OTG5481 et OTG5482 (SEQ ID NO: 16 et 17). Celui-ci est, ensuite, cloné dans le site *Sma*l de M13mp18 pour donner M13TG6519. D'autre part, le vecteur pTG6584 est digéré par *Xba*l puis religué afin d'éliminer le fragment correspondant de la région E3. On obtient pTG6589, lequel est clivé par *Bam*HI, traité à la Klenow puis digéré par *Bst*EII. On introduit dans le vecteur ainsi traité le fragment *Eco*RI (Klenow)-*Bst*EII purifié de M13TG6519, pour générer pTG6590.

A titre indicatif, le vecteur pTG6584 est un vecteur pUC19 (Gibco BRL) qui contient les séquences d'Ad5 s'étendant du site unique *Spe*l (position 27082) jusqu'au début de la région promotrice de la région E4 (position 35826). Il est obtenu par digestion de pTG1659 (exemple 2.3) par *Sal*l et *Spe*l, traité à l'ADN polymérase Klenow puis religation.

### 2. Construction d'un vecteur adénoviral délété de la région E1 et de la partie de E3 n'exprimant pas la protéine gp19Da

La partie de la région E3 de l'Ad5 codant pour la gp19kDa (nucléotides 28731 à 29217) est obtenue par PCR à partir d'une préparation d'ADN génomique d'Ad5 et en mettant en oeuvre les amorces OTG5455 et OTG5456 (SEQ ID NO: 18 et 19). Le fragment généré est introduit dans le site *Sma*l de M13mp18 pour donner M13TG6520. On isole le fragment *Eco*RI-*Xba*l de ce dernier, que l'on clone dans le site *Aaf*ll de pTG1670 (exemple 2.3), les sites ayant été rendus francs par traitement à l'ADN polymérase Klenow. Puis, le fragment *Xba*l purifié du vecteur de l'étape précédente est inséré dans le site *Xba*l du vecteur pTG6590ΔE3 (exemple 3.1.).

### 3. Obtention de particules adénovirales.

Les particules virales recombinantes sont obtenues par ligation des fragments *Spe*l isolés de l'ADN génomique d'AdTG6303 ou AdTG6581 et de l'un ou l'autre des vecteurs des exemples 3.1 et 3.2. Puis, le mélange de ligation est transfecté dans une lignée de complémentation pour la fonction E1.

### EXEMPLE 4 : Construction d'un adénovirus délété des régions E1 et E4.

On amplifie les parties du génome adénoviral s'étendant des nucléotides 31803 à 32799 et 35827 à 35935 à partir d'une préparation d'ADN génomique d'Ad5 et des amorces OTG5728 et OTG5729 (SEQ ID NO: 20 et 21) et OTG5730 et OTG5481 (SEQ ID NO: 22 et 16) respectivement. Après une dizaine de cycles d'amplification, la réaction est poursuivie à partir d'une aliquote des deux mélanges réactionnels en mettant en oeuvre les oligonucléolides OTG5728 et OTG5781. Le fragment amplifié s'étend des nucléotides 31803 à 35935 avec une délétion de l'intégralité de la région E4 (positions 32800 à 35826). Après digestion par *Eco*RI et *Hind*lll*,* il est cloné entre les mêmes sites de M13mp18 pour donner M13TG6521.

M13TG6521 est digéré par *E*coRI, traité à l'ADN polymérase klenow puis clivé par *Bsf*Xl. Le fragment de 0,46 kb comportant l'ITR 3' est inséré entre le site *Bam*HI rendu franc par traitement à l'ADN polymérase klenow et le site *BstX*l de pTG6584 (exemple 3.1). On obtient pTG6587, qui est digéré par *Xb*al puis religué sur lui-même, pour donner pTG6588 (délétion de E3).

On introduit dans le site *Pac*l de pTG6588 un fragment d'ADN synthétique provenant de la réassociation des oligonucléotides OTG6060, OTG6061, OTG6062 et OTG6063 (SEQ ID NO: 23 à 26). Il résulte pTG8500 dans lequel les signaux de terminaison de la transcription des gènes tardifs L5 sont améliorés.

On génère une particule adénovirale (AdΔE4) dont le génome est délété de l'intégralité de la région E4 (nucléotides 32800 à 35826) et du fragment *Xba*l de la région E3 (nucléotides 28592 à 30470), par ligation des fragments *Spe*l isolés de pTG8500 ou pTG6588 et de l'Ad5. Le mélange de ligation est transfecté dans une lignée cellulaire de complémentation pour la fonction E4, par exemple la lignée W162 (Weinberg et Ketner, 1983, Proc. Natl. Acad. Sci. USA, 80, 5383-5386). On obtient un adénovirus défectif pour les fonctions E1 et E4 (ΔE1, ΔE4) par transfection dans une lignée de complémentation pour E1 et E4 (par exemple la lignée de l'exemple 8) du mélange de ligation entre le génome de l'Ad dl324 et le plasmide pTG8500 ou pTG6588 linéarisé par *Spe*l.

D'autre part, on peut également procéder de la manière suivante. On clone la fragment *Spe*l-*Sca*l Isolé de pTG1659 (exemple 2.3) dans le vecteur pTG6588 clivé par ces mêmes enzymes, pour obtenir pTG6591. Ce dernier comporte les séquences de l'Ad5 des nucléotides 21062 à 35935 mais, comme précédemment, délétées de l'intégralité de la région E4 et du fragment *Xba*l de la région E3. On introduit dans le vecteur pTG6591 digéré par *Pac*l, le fragment d'ADN synthétique décrit ci-dessus et on génère pTG6597. Les particules adénovirales peuvent être obtenues par recombinaison homologue entre l'ADN génomique de l'Ad dl324 clivé par *Spe*l et les plasmides pTG6591 ou pTG6597 clivé par *Bam*HI.

### EXEMPLE 5: Construction d'un virus "minimum"

Un vecteur adénoviral dit "minimum" est constitué par clonage dans un plasmide des éléments suivants :
- l'ITR 5' de l'Ad5 (des nucléotides 1 à 103) ;
- la région d'encapsidation de l'Ad5 (des nucléotides 104 à 458) ;
- une séquence nucléotidique exogène comprenant:
   * un premier gène d'intérêt thérapeutique placé de préférence sous le contrôle de son propre promoteur afin d'obtenir une régulation de l'expression la plus proche possible de la régulation naturelle,
   * un deuxième gène d'intérêt constitué du gène TK-HSV-1, et
   * de manière facultative, des séquences nucléotidiques quelconques ajoutées pour des raisons d'efficacité de replication ou d'encapsidation de manière à ce que la taille totale du génome à encapsider soit comprise entre 30 et 36kb;
   * les séquences codant pour la protéine Gal4 de *Saccharomyces cerevisiae* (Laughon et Gesteland, 1964, MoL Cell. Biol., *4*, 260-267) placées sous le contrôle d'un promoteur fonctionnel dans une cellule eucaryote supérieure; et
- l'ITR 3' de l'Ad5 (des nucléotides 35833 à 35935).

L'assemblage de ces différents éléments est réalisé selon les techniques standards de biologie moléculaire. L'obtention de virions infectieux comprenant un tel vecteur se fait comme décrit précédemment dans une lignée de complémentation de l'exemple 7.

### EXEMPLE 6: Constitution d'une cellule de complémentation capable de complémenter en trans la fonction E1.

### 1. Constitution d'une cellule de complémentation comprenant la région E1 des nucléotides 100 à 5297 (pTG6533)

Celle-ci comporte:
- une cassette d'expression du gène pac, lequel est placé sous le contrôle du promoteur précoce du virus SV40 (nucléotides 5171 à 5243) et comprend en 3' le signal de terminaison de la transcription de SV40 (nucléotides 2543 à 2618), Le gène pac utilisé correspond à un fragment allant du nucléotide 252 au nucléotide 905 de la séquence divulguée par Lacalle et al. (1989, Gene, *79*, 375-380) et comportant 4 mutations par rapport à la séquence publiée (C en position 305 remplacé par A ; C en position 367 remplacé par T ; insertion d'un G en position 804 ; délétion d'un G en position 820),
- un fragment du génome de l'Ad5 allant des nucléotides 100 à 5297. Ce fragment comprend les régions E1A et E1B munies de leur propre promoteur et de leur signal de terminaison de la transcription ainsi qu'une fraction de la région E2, recouvrant ainsi les séquences codant pour la protéine IX - A titre indicatif, il semble que la lignée 293 ne soit pas capables de produire une protéine IX fonctionnelle.

La construction est réalisée en plusieurs étapes détaillées ci-après. Le vecteur p polyIII-I* (Lathe et al., 1987. Gene, *57*, 193-201) est soumis à une digestion par les enzymes *Acc*l et *Eco*RI. On clone dans le vecteur ainsi traité le fragment *Eco*RI*-Cla*l isolé du plasmide pTG6164. On obtient le vecteur pTG6528.

Le plasmide pTG6164 est issu de pLXSN (Miller D, 1989, Bio/Techniques, 7, 980) et comprend le gène pac placé sous le contrôle du promoteur précoce du virus SV40. Brièvement, le fragment *Hind*III-*Kpn*I de pLXSN est introduit dans M13TG131 pour produire M13TG4194. On insère dans ce dernier, digéré par *Nhe*l et *Kpn*l, le fragment *Nh*el-*Kpn*l de pMPSV H2 K IL2R (Takeda et al., 1988, Growth Factors, *1*, 59-66) pour produire M13TG4196. Celui-ci est digéré par *Hind*III*-Kpn*l et on clone le fragment issu d'une digestion *Hind*III et d'une digestion partielle *Kpn*l et purifié de pLXSN. On obtient pTG5192. Ce dernier est digéré par *Hind*III et partiellement par *Nh*el et on introduit le fragment *Hind*III-*Nh*el de pBabe Puro (Land et al., 1990, Nucleic Acids Res., *18*, 3587), donnant lieu à pTG6164.

Le vecteur pTG6528 est digéré par *Psf*l et on introduit au niveau de ce site le fragment *Pst*l isolé de pTG6185 (exemple 2-1) comportant le signal de terminaison de la transcription de SV40. On obtient pTG6529. Ce dernier est soumis à une digestion *Eco*RI*-Hpa*l et ligué à deux fragments, d'une part un fragment *Bsp*EI*-Bcg*l (positions 826 à 5297) purifié de l'ADN génomique d'Ad5 et d'autre part un fragment généré par PCR aux extrémités *Eco*RI et *Bsp*EI, pour donner pTG6531. Le fragment PCR est généré par amplification génique à partir de l'ADN génomique d'Ad5 et des amorces OTG4564 et OTG4565 (reporté dans les SEQ ID NO: 27 et 28). Le fragment amplifié est digéré par les enzymes *Eco*RI et *Bsp*EI et mis en ligation comme indiqué dans le paragraphe précédent.

Le vecteur pTG6531 comprend les 2 unités de transcription (celle de la région E1 et celle du gène pac) dans la même orientation. Pour éviter des interférences au niveau de la transcription, on les place dans une orientation tête bêche (réverse l'une par rapport à l'autre) en traitant le pTG6531 par *Bam*HI et en religant. Le vecteur pTG6533 correspond à un clone présentant l'orientation reverse des deux unités.

Le vecteur pTG6533 est transfecté dans une lignée cellulaire mammifère, par exemple la lignée Vero (ATCC, CCL81) ou A549 (ATCC, CCL185) par la technique au phosphate de calcium. Les cellules transfectées sont cultivées selon les recommandations du fournisseur et sont placées 24 heures après la transfection en milieu sélectif contenant de la puromycine (concentration 6 µg/ml). On sélectionne les clones résistants sur lesquels on évalue l'expression des gènes de la région E1 afin de déterminer le clone le plus producteur, qui pourra être utilisé à titre de lignée de complémentation pour la préparation d'un adénovirus défectif pour la fonction E1, tel que celui détaillé dans l'exemple 2.

On analyse l'expression des séquences codant pour les protéines précoces de la région E1 par Northern blot en utilisant des sondes appropriées marquées à l'isotope ³²P. La production des protéines codées par la région E1A est détectée par immunoprécipitation après marquage des cellules à l'isotope ³⁵S et à l'aide d'un anticorps commercial (Oncogène Science Inc., référence DP11).

On peut également vérifier la capacité des produits d'expression de la région E1A à activer le promoteur de la région E1B (par analyse des ARNm E1B par Northern blot) ou à activer le promoteur de la région E2 (par dosage de l'activité enzymatique après transfection transitoire d'un plasmide "reporteur" comprenant le gène CAT (Chloramphénicol Acétyl Transférase) placé sous le contrôle du promoteur E2).

Enfin, on peut infecter ces cellules par l'Ad-RSV-βgal (Stratford-Perricaudet et al., 1992, *supra*) et titrer le virus par la technique agar dès qu'on observe un effet cytopathique. En général, on procède de la façon suivante : les cellules sont infectées à une moi (multiplicité d'infection) de 10. Environ 48 heures après l'infection, l'effet cytopathique étant visible, on lyse les cellules et on dose l'activité β-galactosidase selon le protocole conventionnel (voir par exemple Maniatis et al., 1989, *supra).* Les clones positifs sont réinfectés à une moi plus faible. 48 heures après l'infection, on récolte le surnageant et les cellules selon les techniques classiques. On détermine le titre viral par la méthode sous agar en utilisant des cellules 293. Le rapport du titre obtenu sur le titre de départ constitue le facteur d'amplification.

### 2 Construction d'une lignée cellulaire de complémentation comprenant la région E1 des nucléotides 505 à 4034 (pTG6557, pTG6558, pTG6559, pTG6564 et pTG6565)

Les vecteurs pTG6557, pTG6558 et pTG6559 comprennent :
(i) une cassette d'expression du gène pac (nucléotides 252 à 905 comme précédemment) sous le contrôle :
   - du promoteur E2A de l'Ad2 (nucléotides 27341 à 27030) (dans pTG6558),
   - du promoteur E2A de l'Ad2 délété des séquences comprises entre les nucléotides 27163 à 27182 (pour pTG6557). Une telle mutation permet de diminuer le niveau de base du promoteur E2A, sans affecter l'inductibilité par la protéine trans-activatrice codée par E1A, ou
   - du promoteur précoce SV40 pour pTG6559.
      Dans les trois cas, elle comporte également en 3' le signal de terminaison de la transcription du virus SV40 (nucléotides 2543 à 2618) ; et
(ii) une cassette d'expression comportant la partie de la région E1 de l'Ad5 allant des nucléotides 505 à 4034. Cette portion du génome adénoviral contient l'intégralité des séquences codant pour les protéines précoces de la région E1A, le signal de terminaison de la transcription de l'unité E1A, le promoteur E1B (inductible par la protéine trans-activatrice codée par E1A) et l'intégralité des séquences codantes de la région E1B. Elle inclut également les séquences codant pour la protéine IX, qui chevauchent la région E1B. Cependant, elle est dépourvue du promoteur de la région E1A et du signal de terminaison de la transcription des unités transcriptionnelles E1B et IX. Afin de permettre l'expression des séquences de la région E1, on introduit en 5' du fragment adénoviral, le promoteur du gène murin PGK et en 3' le signal de terminaison de la transcription du gène β-globine de lapin (nucléotides 1542 à 2064 de la séquence divulguée dans la banque de donnée Genebank sous la référence K03256).

De manière facultative, on peut également introduire des séquences nucléotidiques quelconques, par exemple isolées de pBR322 (Bolivar et al., 1977, Gène, *2*, 95-113), entre les cassettes d'expression du gène pac et de la région E1 ; afin d'éviter d'éventuelles interférences transcriptionnelles.

La construction de ces vecteurs s'effectue en plusieurs étapes reportées ci-dessous.

En premier lieu, on amplifie par PCR, la partie du génome de l'Ad5 allant du nucléotide 505 au nucléotide B26 à partir d'une préparation génomique et des amorces OTG5013 qui comprend en 5' un site *Pst*l utile pour les étapes de clonage ultérieures (SEQ ID NO: 29) et OTG4565 chevauchant le site *Bsp*EI (SEQ ID NO: 28). Le fragment généré par PCR, est traité à l'ADN polymérase Klenow puis introduit dans le site *Sma*I de M13mp18 donnant lieu à M13TG6512. La séquence du fragment PCR est vérifiée.

Le vecteur pTG6533 (exemple 6.1) est digéré par les enzymes *Eco*RI et *Bsp*E1. On ligue le vecteur ainsi traité avec, d'une part, le fragment *Pst*l*-Bsp*E1 isolé de M13TG6512 et, d'autre part, le fragment *Eco*RI-*Ps*tl isolé de pKJ-1. Ce dernier comprend la portion du promoteur du gène murin PGK, située entre les nucléotides -524 et -19, dont la séquence est reportée dans Adra et al. (1987, Gene, *60*, 65-74),. Cette étape donne lieu au pTG6552 et permet d'insérer le promoteur du gène murin PGK en amont de la région E1 de l'Ad5 débutant au nucléotide 505.

Par ailleurs, le fragment *Xho*I-*Bam*HI, dont l'extrémité générée par *Xho*l est rendue franche suite au traitement par l'ADN polymérase Klenow, est purifié de pBCMG Neo (Karasuyama et al., 1989, J. Exp. Med., *169*, 13-25). Ce fragment, qui comprend le signal de terminaison de la transcription du gène β-globine de lapin, est introduit entre les sites *Sma*I et *Bam*HI du vecteur p polyll-Sfi/Not-14*(Lathe et al., 1987, Gene, *57*, 193-201). Le vecteur pTG6551 qui résulte, est quant à lui digéré par les enzymes *Sph*I et *Eco*RV afin d'y insérer un fragment du génome de l'Ad5 allant du nucléotide 3665 au nucléotide 4034. Ce fragment est généré par PCR selon le protocole standard. On utilise une préparation d'ADN génomique d'Ad5 à titre de matrice et les amorces OTG5015 qui recouvre le site interne *Sph*I en position 3665 (SEQ ID NO: 30) et OTG 5014 comprenant en 5' un site *Bgl*l (SEQ ID NO: 31).

Le fragment PCR est traité par l'ADN polymérase Klenow avant d'être cloné dans le site *Sma*l de M13mp18, générant M13TG6516. Après vérification de sa séquence, le fragment PCR est resorti par digestion par *Bg*lll, traitement à l'ADN polymérase Klenow et digestion par *Sph*l. Il est inséré entre les sites *Sph*l et *Eco*RV de pTG6551. Il en résulta pTG6554.

D'autre part, le vecteur pTG6529 (exemple 6.1) est soumis à une digestion par les enzymes *Hpa*l et *Hind*lll*.* On purifie le fragment de 2,9 kb comportant le gène pac suivi du signal de terminaison de la transcription du virus SV40. Celui-ci est ligué au fragment *Sma*l-*Hind*lll isolé de pE2 Lac (Boeuf et al., 1990, Oncogene, *5*, 691-699) qui porte le promoteur E2A de l'Ad2. On obtient le vecteur pTG6556. De manière alternative, il peut être ligué au fragment *Sma*l-*Hind*lll isolé de pE2 Lac D9170 (Zajchowski et al., 1985, EMBO J., *4*, 1293-1300) qui porte le promoteur E2A muté de l'Ad2. On obtient, dans ce cas, pTG6550.

pTG6556 est digéré par les enzymes *Eco*RI et *Bam*HI. On insère entre ces sites, le fragment *Eco*RI-*Sac*l isolé de pTG6552 et le fragment *Sac*l-*Bam*HI isolé de pTG6554. On obtient le vecteur pTG6558. La même étape réalisée sur pTG6550 et pTG1643 (exemple 7.1) génère pTG6557 et pTG6559 respectivement.

pTG6557 et pTG6558 sont digérés par *Eco*RV, site unique situé entre les deux cassettes d'expression (gène pac et région E1). On clone dans ce site, un fragment *Eco*RV-*Pvu*ll de 1,88kb isolé de pBR322 (Bolivar et al., *supra),* afin d'éloigner les deux promoteurs. On génère respectivement pTG6564 et pTG6565.

Les vecteurs pTG6557, pTG6558, pTG6559, pTG6564 et pTG6565 sont transfectés dans la lignée cellulaire A549. Comme précédemment, on sélectionne les clones résistant à la puromycine et on vérifie l'expression de la région E1. Les clones exprimant E1 sont destinés à amplifier et propager des adénovirus défectifs pour la fonction E1. La production des produits d'expression de E1 s'accompagne d'un effet cytotoxique mais l'analyse par Southern ne permet pas de mettre en évidence des réarrangements de vecteurs. Après infection par l'Ad-RSV -βgal, plusieurs clones sont capables d'amplifier le virus d'un facteur supérieur à 100.

### 3. Construction d'une cellule de complémentation inductible par la protéine Gal4 de Saccharomyces cerevisiae

Ces vecteurs comprennent comme précédemment la partie de la région E1 de l'Ad5 allant du nucléotide 505 à 4034. Cependant l'expression des séquences de la région E1A est placée sous le contrôle d'un promoteur inductible constituée d'une part du promoteur minimal MLP d'Ad2 (TATA box et signal d'initiation de la transcription ; nucléotides-34 à +33) et d'autre part d'une séquence d'activation du gène Gal 10 activable par la protéine Gal4. La séquence consensus d'activation de 17 nucléotides (17MX), qui correspond au site de fixation de Gal4 est spécifiée dans Webster et al. (1988. Cell, *52*, 169). Le signal de terminaison de la transcription du gène de la β-globine de lapin est placé en 3' de l'unité transcriptionnelle E1B.

On synthétise un premier fragment d'ADN comprenant un dimère de la séquence 17MX (SEQ ID NO: 32 et 33) suivi du promoteur minimal MLP d'Ad2 et muni en son extrémité 5' d'un site *Sal*l et en son extrémité 3' d'un site *Bam*HI. Le site *Sal*l est rendu franc par traitement à l'ADN polymérase klenow. Par ailleurs, on synthétise un second fragment d'ADN comprenant un pentamère de la séquence suivi du même promoteur et muni en 5' et 3' des sites *Xba*l et *Bam*HI. Après digestion par *Xba*l, l'extrémité est rendue franche par traitement à la Klenow polymérase.

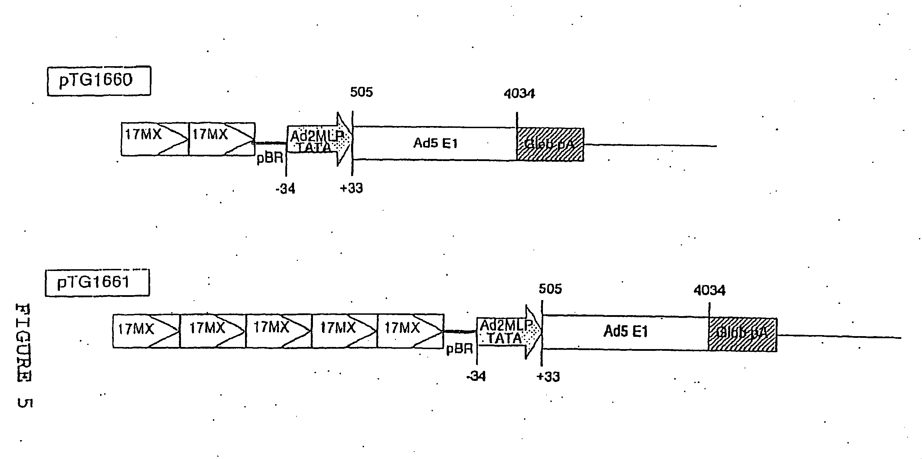

Chacun de ces fragment est introduit dans le site *Bgl*ll de p poly II pour générer respectivement pTG1656 et pTG1657. Puis on introduit dans chacun des vecteurs préalablement digérés par *Pst*l-*Bam*HI, les deux fragments suivants : le fragment *Pst*l-*Xba*l isolé de pTG6552 (Exemple 6.2) et le fragment *Xba*l-*Bam*HI isolé de pTG6559 (exemple 6.2). On obtient pTG1660 et pTG1661 respectivement (Figure 5).

Les cellules A549 sont co-transfectées avec pTG1643 (vecteur d'expression du gène pac) et soit pTG1660 soit pTG1661. Les clones sont sélectionnés pour leur résistance à la puromycine et étudiés comme indiqué précédemment. Environ 50% des clones A549-1660 et A549-1661 produisent des produits d'expression de la région E1. Cependant, la production s'accompagne d'un effet cytotoxique, modifiant l'aspect morphologique des cellules.

L'intégration et le non réarrangement des plasmides dans le génome cellulaire est vérifié par Southern. Aucune modification substantielle des plasmides intégrés (pTG1643, pTG1660 et pTG1661) ne peut être mise en évidence dans les clones producteurs analysés. On peut également vérifier l'inductibilité de l'expression des séquences codées par la région E1A en présence de Gal4 (par transformation par un plasmide permettant l'expression constitutive de la protéine Gal4).

Après l'infection de plusieurs clones producteurs par l'Ad-RSV-Bgal à une moi d'environ 2, deux clones A549-1660 sont capables d'amplifier le stock viral d'un facteur supérieur à 100.

### EXEMPLE 7 : Constitution d'une lignée cellulaire de complémentation pour l'ensemble des fonctions essentielles à la réplication d'un adénovirus

On construit un vecteur comprenant l'ensemble du génome adénoviral de l'Ad5 à l'exception de l'ITR 5', l'ITR 3' et la région d'encapsidation.

Le vecteur pTG6528 (exemple 6.1) est digéré par les enzymes *Pst*l et *Bgl*ll entre lesquels on insère un fragment d'ADN synthétisé chimiquement selon le protocole standard constitué des oligonucléotides des OTG5039 et OTG5040 (SEQ ID NO:34 et 35). La séquence des oligonucléotides est conçue de manière à ne pas reconstituer le site de clonage *Pst*l et introduire un site *E*coRV. On obtient pTG1639, lequel est linéarisé par digestion par *E*coRV et ligué à un fragment *Xba*l-*Bam*HI dont les extrémités sont rendues franches par traitement à l'ADN polymérase Klenow. Ce fragment est porteur du signal de terminaison de la transcription du virus SV40. Tout plasmide comportant un signal entouré des sites de restriction adéquates peut être utilisé à cette étape.

Le vecteur pTG1640 ainsi généré, est digéré par *Bam*HI et *Bg*/ll et le fragment porteur de la cassette d'expression du gène pac est introduit dans le site *Bgl*ll du vecteur pPolyll-Sfi/Not-14*. On obtient le pTG1641. Celui-ci est linéarisé par *Not*l et traité à l'ADN polymérase Klenow. On introduit le fragment *Bam*Hl-Sa*l*l de 0,276 kb isolé de pBR322 (Bolivar et al., *supra*)également traité à l'ADN polymérase Klenow. Ceci donne lieu à pTG1643.

Le pTG1643 est linéarisé par *Xho*l et on insère dans ce site un fragment hybride *Xho*l comportant un dimère 17MX suivi du promoteur minimum du gène IK-HSV-1 (nucléotides 303 à 450 de la séquence divulguée dans la banque de donnée Genebank sous la référence V00467 et complétée en 3' d'un site *Xho*l). On obtient le pTG1647 dans lequel la promoteur hybride 2x17MX-TK-HSV-1 est inséré dans la même orientation que la cassette d'expression du gène pac.

Cette construction, pTG1647, sert de vecteur de base pour introduire entre les sites *Pst*l et *Bam*HI un fragment du génome de l'Ad5 allant du nucléotide 505 au nucléotide 35826. Dans un premier temps, le pTG1647 est digéré par *Pst*l et *Bam*HI puis ligué, d'une part, au fragment *Pst*l*-Cla*l de pTG6552 (exemple 6.2) comportant la partie du génome de l'Ad5 des nucléotides 505 à 918 et, d'autre part, au fragment *Cla*l*-Bam*HI (positions 918 à 21562) préparé à partir de l'ADN génomique de l'Ad5. Le vecteur ainsi obtenu, comporte la partie 5' de l'Ad5 à l'exception de l'ITR5' et de la région d'encapsidation.

Par ailleurs, la partie 3' du génome de l'Ad5 est assemblée dans le vecteur ppolyll-Sfi/Not-14*. Ce dernier est linéarisé par *Bam*HI et on introduit la fragment *Bam*HI-*Avr*ll (nucléotides 21562 à 28752) du génome de l'Ad5 et un fragment PCR correspondant aux nucléotides 35463 à 35826 de l'Ad5. Ce dernier est généré à partir de l'ADN génomique de l'Ad5 et des amorces OTG5024 (SEQ ID NO: 36) et OTG5025 (SEQ ID NO: 37) et comporte en 5' un site *Bam*HI. Le vecteur obtenu est digéré par *Avr*ll et on insère le fragment *Avr*ll Isolé de l'ADN génomique de l'Ad5 et s'étendant des positions 28753 à 35462

Le fragment *Bam*HI comportant les séquences adénovirales est introduit dans le site *Bam*HI du vecteur de l'étape précédente comportant la partie 5' du génome adénoviral dépourvu de l'ITR 5' et la région d'encapsidation.

Une lignée cellulaire de complémentation capable de complémenter l'ensemble des fonctions d'un adénovirus défectif est générée par transfection dans une lignée cellulaire, comme A549, selon le protocole décrit dans les exemples précédents.

On peut également procéder en construisant quatre vecteurs comportant la quasi totalité du génome adénoviral qui sera réassemblé sur un seul vecteur lors de l'étape finale.
- pTG1655 correspond au clonage du fragment *Bsp*E1 (nucléotides 826 à 7269) isolé d'une préparation d'ADN génomique d'Ad5, dans le site *Xm*al de p poly II-Sfl/Not.14*.;
- pTG1664 est généré par l'insertion du fragment *Not*l (nucléotides 6503 à 1504) isolé d'une préparation d'ADN génomique d'Ad5, dans le site *Not*l du même vecteur.
- pTG1662 est obtenu par introduction du fragment *Aat*ll (nucléotides 10754 à 23970) isolé d'une préparation d'ADN génomique d'Ad5 dans le site *Aat*ll de p polyll.
- pTG1659 comportant la partie 3' du génome d'Ad5 (exemple 2.3).

Puis on introduit un fragment comportant un système d'expression inductible comme le promoteur décrit à l'exemple 6.3 ou 7 inductible par Gal4 ou un promoteur de l'art antérieur comme le promoteur metallothionéine ou tétracycline. Un tel fragment est placé en amont des séquences 5' de l'Ad5 (nucléotides 505 à 918) dans le vecteur pTG1665 digéré par *Aat*ll et *Cl*al. Enfin, on clone successivement dans le vecteur précédent et aux sites correspondants les fragments *Not*l de pTG1664, *Aatl*l de pTG1662 et enfin *Bam*HI de pTG1659.

Une lignée cellulaire de complémentation est générée par co-transfection du vecteur précédent et de pTG1643 et on isole les clones résistants à la puromycine. Cette lignée est plus particulièrement destinée à amplifier et encapsider les vecteurs adénoviraux de l'exemple 5 défectifs pour les fonctions E1, E2 et E4 et les fonctions tardives.

### EXEMPLE 8: Constitution d'une lignée cellulaire de complémentation pour les fonctions E1 et E4.

Le vecteur pTG1647 (exemple 7) est digéré par les enzymes *Pst*l-*Bam*HI et on introduit dans le vecteur ainsi traité 3 fragments:
- le fragment *Psf*l-*Xba*I de pTG6552 (exemple 6.2) portant les séquences d'Ad5 du nucléotide 505 au nucléotide 1339,
- le fragment *Xba*l-*Sph*l de pTG6552 portant les séquences d'Ad5 du nucléotide 1340 au nucléotide 3665, et
- le fragment *Sph*l-*Bam*HI de pTG6554 (exemple 6.2) portant les séquences d'Ad5 du nucléotide 3665 à 4034 et un signal de terminaison de la transcription.

Le vecteur ainsi obtenu est coupé par *Bam*HI et on introduit dans ce site trois fragments qui sont les suivants :
- un fragment digéré par *Bam*Hl-*Afl*ll généré par PCR correspondant à la séquence de l'Ad5 situé entre les positions 32800 à 33104. On utilise l'ADN génomique d'Ad5 comme matrice et les amorces OTG5078 (SEQ ID NO: 38) et OTG5079 (SEQ ID NO: 39),
- le fragment *Afl*ll-*Avr*ll isolé, de l'ADN génomique d'Ad5 (nucléotides 33105 à 35463),
- le fragment *Avr*ll*-Bam*HI généré par PCR à l'aide des amorces OTG5024 et OTG5025 (voir exemple 7).

Le vecteur ainsi généré est introduit dans une lignée cellulaire selon le protocole décrit précédemment, pour constituer une lignée de complémentation pour les fonctions E1 et E4.

Par ailleurs, une telle lignée peut également être obtenue selon le protocole suivant :

La région E4 du génome de l'Ad5 (nucléotides 32800 à à 35826) est reconstituée en plusieurs étapes. La partie allant des nucléotides 33116 à 32800 est synthétisée par PCR à partir de l'ADN génomique d'Ad5 avec le couple d'amorces OTG5078 et OTG5079 (SEQ ID NO: 38 et 39), puis insérée dans le site *Eco*RV de M73TG130, pour générer M13TG1645.

Le fragment *Bam*HI-*Afl*ll de ce dernier est engagé dans une réaction de ligation avec le fragment *Afl*ll-*Avr*ll d'Ad5 (nucléotides 33104 à 35463) et le vecteur pTG7457 digéré par *Bam*HI et *Avr*ll. On obtient pTG1650.

Puis on complète la région E4 par obtention du fragment correspondant aux nucléotides 35826 à 35457 par PCR à partir d'une préparation d'ADN génomique d'Ad5 et des amorces OTG5024 et OTG5025 (SEQ ID NO: 36 et 37). Celui-ci est inséré dans le site *Sma*l de M13mp18 pour donner M13TG1646. Le fragment *Avr*ll*-Eco*RI est isolé de ce dernier et cloné entre les sites *Avr*ll et *Eco*RI de pTG1650. On obtient pTG1652.

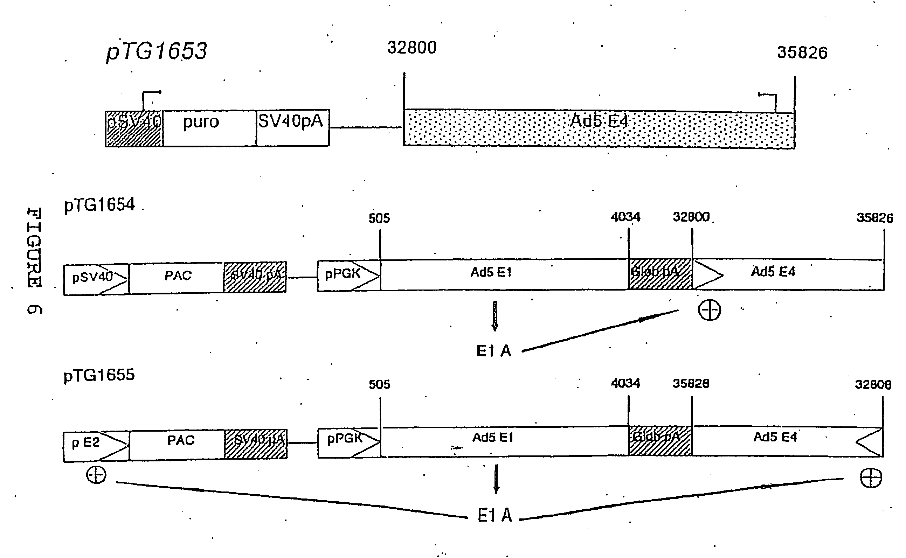

Le fragment *Bam*HI comportant la région E4 d'Ad5 est isolé de pTG1652 et cloné dans le site *Bam*HI de pTG1643, de pTG6559 (exemple 62) ou dans le site Sspl de pTG6564 (exemple 6.2) après avoir rendu les sites francs, pour générer pTG1653, pTG1654 et pTG1655 (Figure 6) respectivement.

On génère par des techniques conventionnelles une cellule de complémentation capable de complémenter *en trans* les fonctions E1 et E4, par:
(1) transformation de pTG1653 dans la lignée cellulaire 293, ou
(2) transformation de pTG1654 ou pTG1655 dans la lignée cellulaire A549.

D'une manière générale, l'expression des produits des régions E1 et E4 s'accompagne d'un effet cytotoxique. Un certain nombre de clones 293-1653 est capable de complémenter à la fois des adénovirus délétés de E1 et des adénovirus délétés de E4.

Une autre alternative consiste à procéder de la manière suivante.

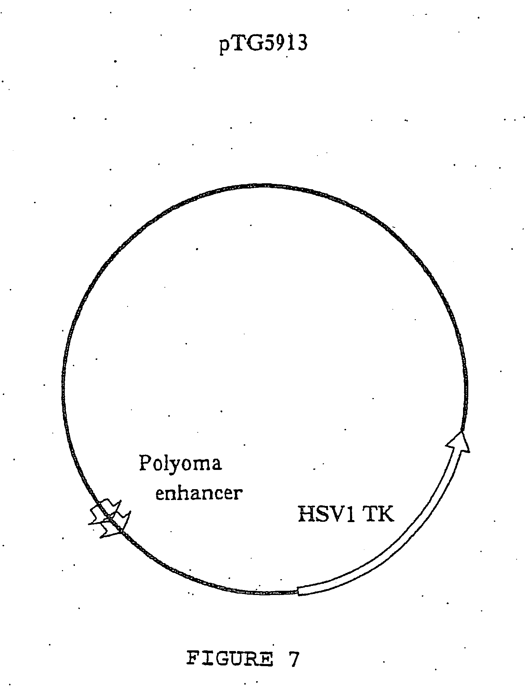

Le vecteur M13TG1646 est soumis à une mutagénèse dirigée avec l'oligonucléotide mutagène OTG5991 (SEQ ID NO: 40) dans le but de déléter le promoteur de la région E4 et d'insérer un site *Hpa*l. Le vecteur muté est désigné M13TG6522. Il est digéré par *Pst*l, traité à l'ADN polymérase du phage T4 puis par *Avr*ll et mis en ligation avec un fragment *Eco*RI (Klenow)-*Avr*ll) purifié de pTG1652 (exemple B), pour donner pTG6595. Ce dernier est clivé par *Hpa*l et on introduit le fragment de 0,8 kb obtenu de pTG5913 (Figure 7) après digestion *Bg*lll et *Bam*HI et traitement à la Klenow. On génère pTG6596 dans lequel la région E4 (positions 32800 à 35826) est placée sous la contrôle du promoteur TK. A titre indicatif, pTG5913 porte le gène TK-HSV-1 et le fragment *Bgl*ll-*Bam*HI correspond au promoteur de ce gène (Wagner et al., 1981, Proc. Natl. Acad. Sci., USA, *78,* 1441 - 1445).

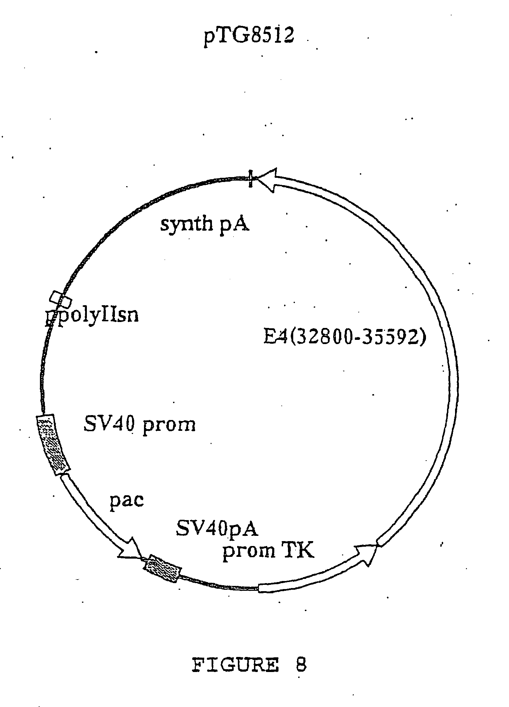

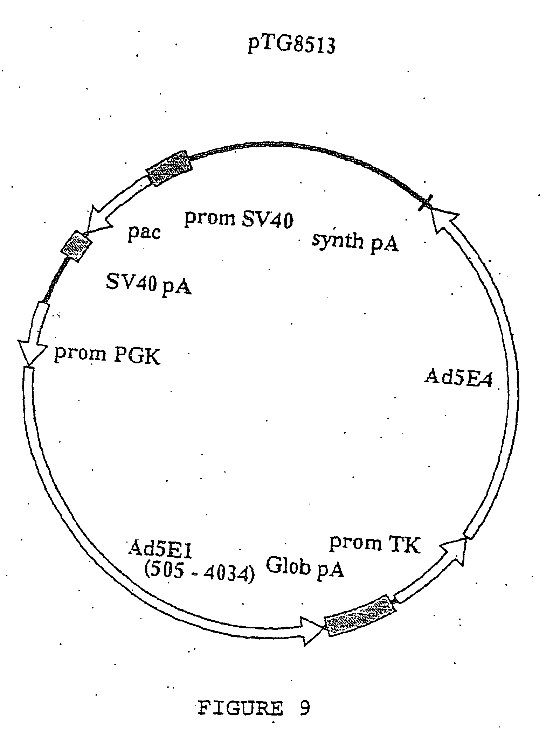

Parallèlement, les vecteurs pTG1643 et pTG6559 (exemple 6) sont linéarisés par *Bam*HI et on insère un fragment synthétique issu de la réassociation des oligonucléotides OTG6141 et OTG6142 (SEQ ID NO: 41 et 42), pour obtenir respectivement pTG8508 et pTG8507. Ces derniers sont clivés par *Bam*HI avant d'introduire le fragment *Bam*HI purifié de pTG6596 comportant la cassette d'expression de E4. On génère les vecteurs pTG8512 (Figure 8) et pTG8513 (Figure 9).

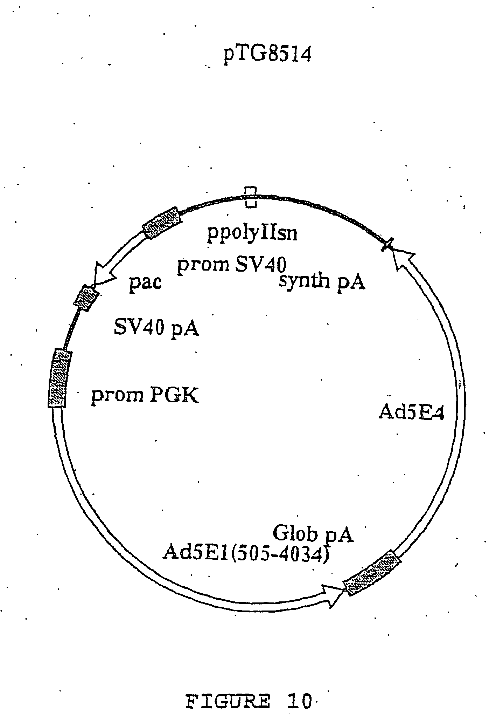

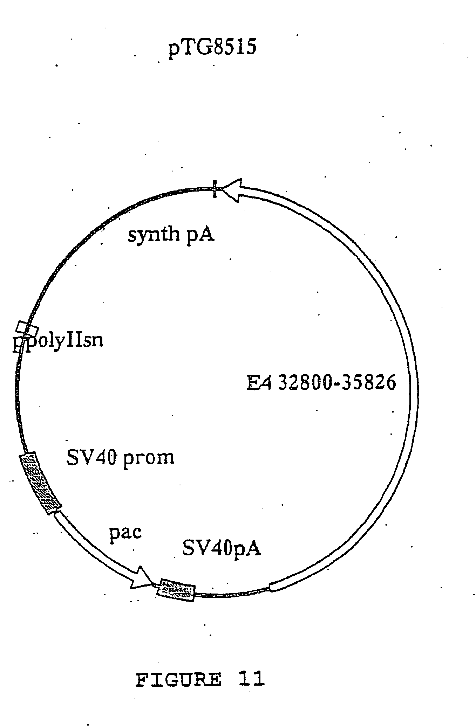

D'autre part, l'introduction du fragment *Bam*HI de pTG1652 dans le vecteur pTG8508 ou pTG8507 linéarisé par la même enzyme aboutit à pTG8514 et pTG8515 respectivement (Figures 10 et 11).

Les lignées cellulaires transfectées par pTG8512 ou pTG8515 permettront de complémenter un adénovirus défectif pour la fonction E4, alors que celles résultant de la transfection de pTG8513 ou pTG8514 sont destinées à amplifier et propager des adénovirus défectifs pour les fonctions E1 et E4. De même, la transfection de pTG8512 ou pTG8515 dans les cellules 293 permettront de complimenter des adénovirus défectifs pour E1 et E4.

## Revendications

1. Une lignée cellulaire de complémentation propre à la production de particules d'adénovirus destinées à un usage thérapeutique ou prophylactique humain ladite lignée cellulaire de complémentation comportant un élément de complémentation; ledit élément de complémentation étant **caractérisé en ce qu'**il comprend comme seul ADN adénoviral la partie de la région E1A codant pour les protéines précoces de la région E1A et l'intégralité des séquences codant pour les protéines précoces de la région E1B placée sous le contrôle de son propre promoteur et **en ce que** l'expression de la partie de la région E1A est placée sous le contrôle d'un promoteur hétérologue; et
ledit élément de complémentation
(a) étant capable de complémenter *en trans* un vecteur adénoviral qui est dérivé d'un adénovirus humain et qui est défectif pour la fonction E1; et
(b) étant intégré dans le génome de ladite lignée cellulaire de complémentation.

2. La lignée cellulaire de complémentation selon la revendication 1, dans laquelle l'adénovirus est un adénovirus humain de type 5.

3. La lignée de complémentation selon le revendication 1 ou 2, dans laquelle la région E1A est placée sous le contrôle d'un promoteur inductible par une protéine trans-activatrice de transcription non-adénovirale.

4. La lignée de complémentation selon la revendication 3, dans laquelle ladite protéine trans-activatrice de transcription non adénovirale est codée par un vecteur adénoviral recombinant, ce vecteur adénoviral étant placé sous le contrôle d'éléments nécessaires à son expression dans une cellule hôte et comportant un gène codant notamment pour une protéine trans-activatrice de transcription Gal4 de *Saccharomyces cerevisiae.*

5. La lignée cellulaire de complémentation selon la revendication 3 ou 4, dans laquelle la région E1A est placée sous le contrôle d'un promoteur inductible par une protéine trans-activatrice de transcription Gal4 de *Saccharomyces cerevisiae.*

6. La lignée cellulaire de complémentation selon l'une des revendications 1 à 5, comprenant en outre un gène codant pour un marqueur de sélection.

7. La lignée cellulaire de complémentation selon la revendication 6, dans laquelle le gène de séléction code pour la puromycine acetyl-transferase.

8. La lignée cellulaire de complémentation selon l'une des revendications 1 à 7, dérivée d'une lignée cellulaire acceptable d'un point de vue pharmaceutique.

9. La lignée cellulaire de complémentation selon la revendication 8, dérivée d'une lignée cellulaire sélectionnée parmi les lignées Vero, A549, MRC5 et W138.

10. La lignée cellulaire de complémentation selon l'une des revendications 1 à 8, dérivée d'une cellule de la rétine d'un embryon humain.

11. La lignée cellulaire de complémentation selon l'une des revendications 1 à 10, **caractérisée en ce que** ledit promoteur hétérologue est le promoteur du gène murin PGK.

12. La lignée cellulaire de complémentation selon la revendication 11, **caracterisée en ce que** ledit élément de complémentation de la fonction E1 est placé sous le contrôle d'un signal de terminaison de la transcription hétérologue, notamment celui du gène β-globine de lapin.

13. Une composition pharmaceutique comprenant à titre d'agent thérapeutique ou prophylactique une lignée cellulaire de complémentation selon l'une des revendications 1 à 12, en association avec un support acceptable d'un point de vue pharmaceutique.

14. Usage d'une lignée cellulaire de complémentation selon l'une des revendications 1 à 12 pour la préparation d'une composition pharmaceutique pour transférer des molécules d'ADN dans une cellule ou un organisme eucaryote.

15. Un procédé de préparation d'une particule d'adénovirus comprenant un vecteur adénoviral défectif pour la réplication dérivant du génome d'un adénovirus par délétion d'au moins tout ou partie de la région E1A selon lequel:
(i) on introduit undit vecteur adénoviral dans une lignée cellulaire de complémentation selon l'une des revendications 1 à 12 capable de complémenter en trans ledit vecteur adénoviral pour obtenir une lignée cellulaire de complémentation transfectée;
(ii) on cultive ladite lignée cellulaire de complémentation transfectée dans les conditions appropriées pour permettre la production de ladite particule d'adénovirus; et
(iii) on récupère ladite particule d'adénovirus dans la culture cellulaire.

## Claims

1. A complementation cell line suitable for the production of adenovirus particles which are intended for human therapeutic or prophylactic use, said complementation cell line containing a complementation element, wherein said complementation element is **characterised in that** it comprises as the only adenovirus DNA the part of the E1A region encoding the early proteins of the E1A region and the entirety of the sequences encoding the early proteins of the E1B region placed under the control of its own promoter and **in that** the expression of said part of the E1A region is placed under the control of a heterologous promoter; and
said complementation element
(a) being capable of complementing *in trans* an adenoviral vector which is derived from a human adenovirus and which is defective for the E1 function; and
(b) being integrated into the genome of the complementation cell line.

2. The complementation cell line according to claim 1, wherein the adenovirus is a type-5 human adenovirus.

3. The complementation cell line according to claim 1 or 2, in which the E1A region is placed under the control of a promoter inducible by a non-adenoviral transcription trans-activator protein.

4. The complementation cell line according to claim 3, in which said non-adenoviral transcription trans-activator protein is encoded by a recombinant adenoviral vector, wherein said adenoviral vector is placed under the control of elements necessary for its expression in a host cell and contains a gene encoding in particular a Gal4 transcription trans-activator protein of *Saccharomyces cerevisiae.*

5. The complementation cell line according to claim 3 or 4 in which the E1A region is placed under the control of a promoter inducible by a Gal4 transcription trans-activator protein of *Saccharomyces cerevisiae.*

6. The complementation cell line according to any one of claims 1 to 5, further comprising a gene encoding a selection marker.

7. The complementation cell line according to claim 6, wherein the selection gene encodes puromycin acetyl-transferase.

8. The complementation cell line according to any one of claims 1 to 7, derived from a pharmaceutically acceptable cell line.

9. The complementation cell line according to claim 8, derived from a cell line selected from the lines Vero, A549, MRC5 and W138.

10. The complementation cell line according to any one of claims 1 to 8, derived from a cell of the retina of a human embryo.

11. The complementation cell line according to any one of the claims 1 to 10, **characterised in that** said heterologous promoter is the promoter of the murine PGK gene.

12. The complementation cell line according to claim 11, **characterised in that** said complementation element for the E1 function is placed under the control of a heterologous transcription termination signal, in particular that of the rabbit β-globin gene.

13. A pharmaceutical composition comprising as therapeutic or prophylactic agent a complementation cell line according to any one of claims 1 to 12 in association with a pharmaceutically acceptable carrier.

14. Use of a complementation cell line according to any one of claims 1 to 12 for the preparation of a pharmaceutical composition for transferring DNA molecules into a cell or a eukaryotic organism.

15. A process for preparing an adenovirus particle comprising an adenoviral vector defective for replication derived from the genome of an adenovirus by deletion of at least all or part of the E1A region, wherein:
(i) the adenoviral vector is introduced into a complementation cell line according to any one of the claims 1 to 12 capable of complementing in trans said adenoviral vector to obtain a transfected complementation cell line;
(ii) said transfected complementation cell line is cultivated under appropriate conditions to permit the production of said adenovirus particle; and
(iii) said adenovirus particle is recovered from the cell culture.

## Patentansprüche

1. Komplementationszelllinie, die zur Herstellung von Adenoviruspartikeln geeignet ist, die für eine therapeutische oder prophylaktische Verwendung beim Menschen bestimmt sind, wobei die Komplementationszelllinie ein Komplementationselement enthält; wobei das Komplementationselement **dadurch gekennzeichnet ist, dass** es als einzige Adenovirus-DNA den Teil der E1A-Region enthält, der die frühen Proteine der E1A-Region codiert, sowie die Gesamtheit der Sequenzen, die die frühen Proteine der E1 B-Region codieren, die unter die Kontrolle ihres eigenen Promotors gestellt ist, und das **dadurch gekennzeichnet ist, dass** die Expression des Teils der E1A-Region unter die Kontrolle eines heterologen Promotors gestellt ist; und
wobei das Komplementationselement
(a) fähig ist, einen Adenovirusvektor *in trans* zu komplementieren, der von einem humanen Adenovirus abgeleitet ist und der für die Funktion E1 defekt ist; und
(b) in das Genom der Komplementationszelllinie integriert ist.

2. Komplementationszelllinie nach Anspruch 1, in der das Adenovirus ein Typ-5-Menschen-Adenovirus ist.

3. Komplementationszelllinie nach Anspruch 1 oder 2, in der die E1A-Region unter die Kontrolle eines Promotors gestellt ist, der durch ein nichtadenovirales Transkriptions-Transaktivierungsprotein induzierbar ist.

4. Komplementationszelllinie nach Anspruch 3, in der das nicht-adenovirale Transkriptions-Transaktivierungsprotein von einem rekombinanten Adenovirusvektor codiert wird, wobei dieser Adenovirusvektor unter die Kontrolle von Elementen gestellt ist, die für seine Expression in einer Wirtszelle erforderlich sind, und ein Gen enthält, das insbesondere ein Gal4-Transkriptions-Transaktivierungsprotein von *Saccharomyces cerevisiae* codiert.

5. Komplementationszellline nach Anspruch 3 oder 4, in dem die E1A-Region unter die Kontrolle eines Promotors gestellt ist, der durch ein Gal4-Transkriptions-Transaktivierungsprotein von *Saccharomyces cerevisiae* induzierbar ist.

6. Komplementationszelllinie nach einem der Ansprüche 1 bis 5, die unter anderem ein einen Selektionsmarker codierendes Gen umfasst.

7. Komplementationszelllinie nach Anspruch 6, wobei das Selektionsgen Puromycinacetyl-Transferase codiert.

8. Komplementationszelllinie nach einem der Ansprüche 1 bis 7, die von einer pharmazeutisch verträglichen Zelllinie abgeleitet ist.

9. Komplementationszelllinie nach Anspruch 8, die von einer aus den Linien Vero, A549, MRC5 und W138 ausgewählten Zelllinie abgeleitet ist.

10. Komplementationszelllinie nach einem der Ansprüche 1 bis 8, die von einer Netzhautzelle eines menschlichen Embryos abgeleitet ist.

11. Komplementationszelllinie nach einem der Ansprüche 1 bis 10, **dadurch gekennzeichnet, dass** der heterologe Promotor der Promotor des murinen PGK-Gens ist.

12. Komplementationszelllinie nach Anspruch 11, **dadurch gekennzeichnet, dass** das Komplementationselement der E1-Funktion unter die Kontrolle eines heterologen Transkriptionsterminationssignals gestellt ist, insbesondere desjenigen des β-Globingens von Kaninchen.

13. Arzneimittel, das als therapeutischen oder prophylaktischen Wirkstoff eine Komplemetationszelllinie nach einem der Ansprüche 1 bis 12 in Verbindung mit einem pharmazeutisch verträglichen Träger enthält.

14. Verwendung einer Komplementationszelllinie nach einem der Ansprüche 1 bis 12 zur Herstellung eines Arzneimittels zum Einbringen von DNA-Molekülen in eine Zelle oder in einen eukaryontischen Organismus.

15. Verfahren zur Herstellung eines Adenoviruspartikels, der einen replikationsdefekten Adenovirusvektor enthält, der durch Deletion von mindestens der gesamten oder einem Teil der E1A-Region vom Genom eines Adenovirus abgeleitet ist, wobei
(i) ein Adenovirusvektor in eine Komplementationszelllinie nach einem der Ansprüche 1 bis 12 eingeführt wird, die fähig ist, den Adenovirusvektor in trans zu komplementieren, um eine transfizierte Komplementationszelllinie zu erhalten;
(ii) die transfizierte Komplementationszelllinie unter Bedingungen gezüchtet wird, die geeignet sind, die Produktion des Adenoviruspartikels zu ermöglichen; und
(iii) das Adenoviruspartikel aus der Zellkultur gewonnen wird.
